# EUROPEAN PATENT SPECIFICATION

(11) **EP 1 515 970 B1**
(45) Date of publication and mention of the grant of the patent: **18.10.2006**
(21) Application number: 03733333.3
(22) Date of filing: 10.06.2003
(51) Int. Cl.: C07D 473/00, C07D 473/30, C07D 473/34, C07H 19/16

(54) **ALKYNYLPURINE COMPOUNDS AND PRODUCTION METHODS THEREOF**
ALKYNYLPURINE UND VERFAHREN ZU DEREN HERSTELLUNG
COMPOSES D'ALKYNYLPURINE ET PROCEDES DE PRODUCTION CORRESPONDANTS

(30) Priority: 14.06.2002 JP 2002175015
(43) Date of publication of application: 23.03.2005
(73) Proprietor: Sumitomo Chemical Company, Limited, Tokyo 104-8260 (JP)
(72) Inventor: HAYASHI, Taketo, c/o Sumitomo Chemical CO., LTD, Osaka-shi, Osaka 555-0021 (JP); KAWAKAMI, Takehiko, c/o Sumitomo Chemical CO.,LTD, Osaka-shi, Osaka 555-0021 (JP); KUMAZAWA, Hiroharu, c/o Sumitomo Chemical CO.,LTD, Osaka-shi, Osaka 555-0021 (JP); KOTSCHY, András, c/o Dept General & Inorganic Chem, H-1117 Budapest (HU); CSÁMPAI, Antal C/o Dep. of Gen. and Inorg. Chem., 1117 Budapest (HU); NAGY, András C/o Dep. of Gen. and Inorg. Chem., 1117 Budapest (HU)
(74) Representative: Duckworth, Timothy John
(86) International application number: PCT/JP2003/007317
(87) International publication number: WO 2003/106458

(56) References cited:
- EP-A- 0 488 336
- EP-A- 1 054 012
- ADAH S A ET AL: "Triflic Enolates In the Palladium-Mediated Synthesis of Complex Ethynyl Adenosines" TETRAHEDRON LETTERS, ELSEVIER SCIENCE PUBLISHERS, AMSTERDAM, NL, vol. 36, no. 36, 4 September 1995 (1995-09-04), pages 6371-6372, XP004027233 ISSN: 0040-4039
- NAIR V ET AL: "STRATEGICALLY FUNCTIONALIZED ADENOSINES: AGONISTS FOR ADENOSINE RECEPTORS" NUCLEOSIDES & NUCLEOTIDES, MARCEL DEKKER, INC, US, vol. 14, no. 3-5, 1995, pages 537-539, XP009017441 ISSN: 0732-8311
- HOCEK M ET AL: "COVALENT ANALOGUES OF DNA BASE-PAIRS AND TRIPLETS. PART 2: SYNTHESIS AND CYTOSTATIC ACTIVITY OF BIS(PURIN-6-YL)ACETYLENES, -DIACETYLENES AND RELATED COMPOUNDS" BIOORGANIC & MEDICINAL CHEMISTRY LETTERS, OXFORD, GB, vol. 12, no. 7, 2002, pages 1055-1058, XP009017440 ISSN: 0960-894X
- HOCEK M ET AL: "COVALENT ANALOGUES OF DNA BASE-PAIRS AND TRIPLETS V+. SYNTHESIS OF PURINE-PURINE AND PURINE-PYRIMIDINE CONJUGATES CONNECTED BY DIVERSE TYPES OF ACYCLIC CARBON LINKAGES" COLLECTION OF CZECHOSLOVAK CHEMICAL COMMUNICATIONS, ACADEMIC PRESS, LONDON, GB, vol. 67, no. 10, 2002, pages 1560-1578, XP009017464 ISSN: 0010-0765
- MAGER P P: "NEURAL NETWORK APPROACHES APPLIED TO SELECTIVE A2A ADENOSINE RECEPTOR AGONISTS" MEDICINAL CHEMISTRY RESEARCH, BIRKHAEUSER, BOSTON, US, vol. 8, no. 6, 1998, pages 277-290, XP000952528 ISSN: 1054-2523
- LEGRAVEREND M ET AL: "Synthesis of C2 alkynylated purines, a new family of potent inhibitors of cyclin-dependent kinases" BIOORGANIC & MEDICINAL CHEMISTRY LETTERS, OXFORD, GB, vol. 8, no. 7, 7 April 1998 (1998-04-07), pages 793-798, XP004136967 ISSN: 0960-894X
- CAMAIONI E ET AL: "NEW SUBSTITUTED 9-ALKYLPURINES AS ADENOSINE RECEPTOR LIGANDS" BIOORGANIC & MEDICINAL CHEMISTRY, ELSEVIER SCIENCE LTD, GB, vol. 6, no. 5, 1998, pages 523-533, XP002926121 ISSN: 0968-0896
- MATSUDA A ET AL: "Nucleosides and nucleotides. 103. 2-Alkyladenosines: a novel class of selective adenosine A2 receptor agonists with potent antihypertensive effects" JOURNAL OF MEDICINAL CHEMISTRY, AMERICAN CHEMICAL SOCIETY. WASHINGTON, US, vol. 35, 1992, pages 241-252, XP002170995 ISSN: 0022-2623
- BAKKESTUEN A K ET AL: "9-Benzylpurines with inhibitory activity against Mycobacterium tuberculosis" BIOORGANIC & MEDICINAL CHEMISTRY LETTERS, OXFORD, GB, vol. 10, no. 11, June 2000 (2000-06), pages 1207-1210, XP004200557 ISSN: 0960-894X

## Description

### Technical Field

The present invention relates to an alkynylpurine compound (particularly, an alkynylpurine compound having an alkynyl moiety at the 2-, 6- or 8-position of its purine nucleus), which is a key intermediate for the production of medicaments, a salt thereof and a production method thereof.

### Background Art

Alkynylpurine compound is a key intermediate for the production of medicaments such as ischemia-reperfusion injury inhibitors (JP-A-2001-64183), therapeutic agents of diabetes (WO99/35147), therapeutic agents of tuberculosis (A.K. Bakkestuen et al., Bioorg. Med. Chem. Lett., 10 (2000), 1207-1210) and the like.

As a production method of the alkynylpurine compound, for example, the methods described in the following references (1)-(3) can be shown.
(1) According to the method described in "Regiochemistry in the Pd-Mediated Coupling between 6,8-Dihalopurines and Organometallic Reagents", Acta Chemica Scandinavica 53 (1999), 366-372 by Nolsϕe et al., alkynylpurine compounds can be obtained by reacting a 6,8-dihalogenopurine compound 1a-1e with Ph-C≡C-SnBu₃ in the presence of bis (triphenylphosphine) palladium dichloride [i. e. (Ph₃P)₂PdCl₂] or tetrakis[tri(2-furyl)phosphine]palladium [i.e. [(2-furyl)₃P]₄Pd]. 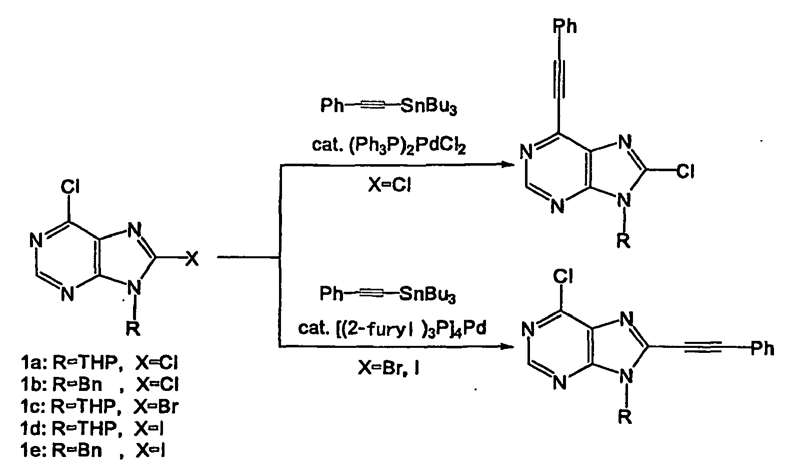 wherein THP is tetrahydropyranyl and Bn is benzyl.
(2) According to the method described in "9-Benzylpurines with Inhibitory Activity Against *Mycobacterium tuberculosis",* Bioorg. Med. Chem. Lett., 10 (2000) 1207-1210 by A.K. Bakkestuen et al., alkynylpurine compounds useful as intermediates for medicaments can be obtained by reacting a halogenopurine compound 1a-1f with Ph-C≡C-SnBu₃ in the presence of bis(triphenylphosphine)palladium dichloride [i.e. (Ph₃P)₂PdCl₂] . 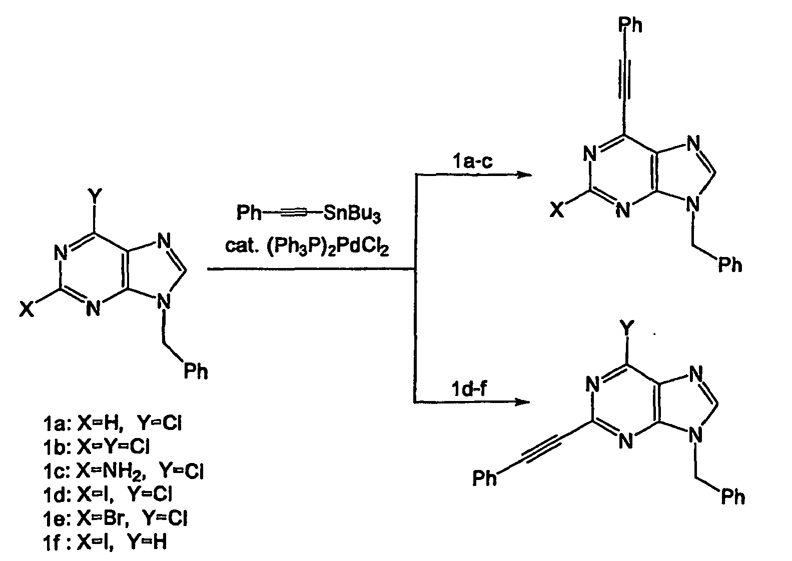
(3) According to the method described in EP 0488336 A1, alkynylpurine compounds useful as medicaments can be obtained by reacting a halogenopurine compound with an alkyne compound in the presence of a palladium catalyst (e.g., bis(triphenylphosphine)palladium dichloride etc.) and a copper compound (e.g., cuprous iodide etc.) according to the following reaction scheme: 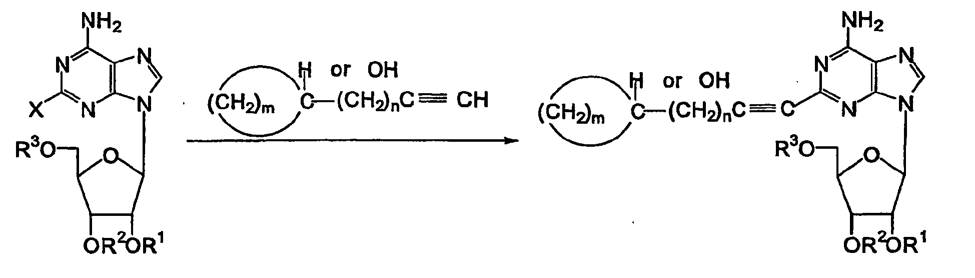
wherein R¹, R² and R³ may be the same or different and each independently represent a hydrogen atom, a hydroxy-protecting group or a phosphoric acid residue, X represents bromine or iodine, m is an integer of 2 to 7 and n is 0 or an integer of 1 to 3.

The methods described in references (1) and (2) both utilize a coupling reaction (Stille reaction) between Ph-C≡C-SnBu₃ (alkynylating agent), which is stable as a metal acetylide, and a halogenopurine by the use of a palladium catalyst. However, the alkynylating agent has extremely high toxicity, because it is an organotin reagent, and the use of the alkynylating agent for the production of medicaments is not preferable. In addition, the methods described in references (1) and (2) are also associated with the problem that the production cost of alkynylpurine compound becomes high because the synthesis of the alkynylating agent is extremely difficult.

Moreover, the above-mentioned methods require Ph-C≡C-SnBu₃ in an amount of at least 1 equivalent, generally not less than 1 equivalent, relative to a halogenopurine compound, which is a substrate, and organotin compounds, which are to be wasted after the reaction, are produced in an amount of not less than 1 equivalent. Therefore, the above-mentioned methods are not environmentally or economically superior.

In addition, inasmuch as the methods described in references (1), (2) and (3) are based on an idea that a halogenopurine compound and an alkynylating agent or alkynyl compound are coupled for each objective product, further modification of the alkynyl moiety of the alkynylpurine compound obtained according to the method described in references (1), (2) or (3) is not assumed. Therefore, the methods described in references (1), (2) and (3) are problematic in that many alkynylpurine derivatives useful as a medicament cannot be synthesized conveniently from the obtained alkynylpurine compounds, and the like.

Accordingly, the development of a method of producing an alkynylpurine compound useful as an intermediate for the production of medicaments (i.e., alkynylpurine compound whose alkynyl moiety can be easily modified, and as a result, a number of alkynylpurine derivatives can be derived) safely, conveniently and economically from the corresponding purine compound, as well as the development of an alkynyl purine compound useful as an intermediate for medicament production are desired.

### Disclosure of the Invention

It is therefore an object of the present invention to provide a production method of an alkynylpurine compound useful as an intermediate for the production of medicaments and a salt thereof safely, conveniently and economically from the corresponding purine compound and a salt thereof, as well as an alkynylpurine compound useful as an intermediate for the production of medicaments and a salt thereof.

In view of the above-mentioned problems, the present inventors have conducted intensive studies and, as a result, found that an ethynylpurine compound having an ethynyl group as an alkynyl moiety can be converted to various alkynylpurine compounds, as a result of which found a method that can produce a purine compound having an alkynyl moiety at the 2-, 6- or 8-position (i.e., alkynylpurine compound whose alkynyl moiety can be easily modified and can derive a number of alkynylpurine derivatives), which is a key intermediate for medicaments, safely, conveniently and economically, which resulted in the completion of the invention.

Accordingly, the present invention relates to the following [1] - [23].
[1] A compound represented by the formula (I-1): 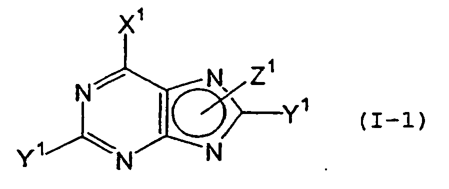 wherein
   - X¹: is an alkyl group, an alkoxy group, an aryl group, an optionally protected amino group, a halogen atom or a hydrogen atom,
   - one of Y¹: is a group represented by the formula: R-C≡C-wherein R is a hydrogen atom, and the other Y¹ is a hydrogen atom, and
   - Z¹: is an alkyl group, an amino-protecting group or a hydrogen atom, which is attached to a nitrogen atom at the 7- or 9-position of the purine nucleus,
   or a salt thereof.
[2] The compound of the above-mentioned [1], wherein X¹ is a halogen atom, and Z¹ is an amino-protecting group or a hydrogen atom.
[3] The compound of the above-mentioned [1] or [2], wherein X¹ is a chlorine atom.
[4] The compound of any of the above-mentioned [1] to [3], wherein Z¹ is tetrahydropyran-2-yl, benzyl or a hydrogen atom.
[5] A compound represented by the formula (I-2): 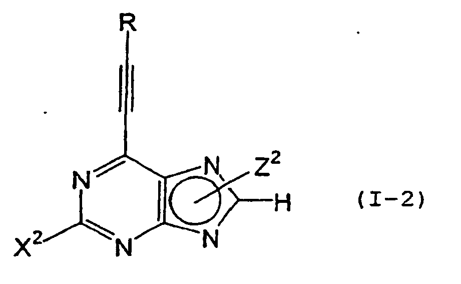 wherein
   - R: is a hydrogen atom or a hydrocarbon group optionally having substituents,
   - X²: is an alkyl group, an alkoxy group, an aryl group, an optionally protected amino group or a halogen atom, and
   - Z²: is an alkyl group, an amino-protecting group or a hydrogen atom, which is attached to a nitrogen atom at the 7- or 9-position of the purine nucleus,
   or a salt thereof.
[6] The compound of the above-mentioned [5], wherein R is a hydrogen atom or Me₂(OH)C-, X² is an optionally protected amino group, and Z² is an amino-protecting group or a hydrogen atom.
[7] The compound of the above-mentioned [5] or [6], wherein Z² is tetrahydropyran-2-yl, benzyl or a hydrogen atom.
[8] A production method of a compound represented by the formula (I-1"): 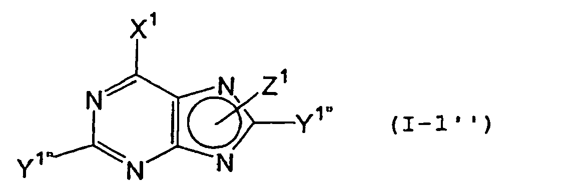 wherein
   X¹ is an alkyl group, an alkoxy group, an aryl group, an optionally protected amino group, a halogen atom or a hydrogen atom,
   Z¹ is an alkyl group, an amino-protecting group or a hydrogen atom, which is attached to a nitrogen atom at the 7- or 9-position of the purine nucleus, and one of Y^{1"} is a group represented by the formula: HC≡C-, and the other Y^{1"} is a hydrogen atom, or a salt thereof, which comprises treating a compound represented by the formula (I-1'): 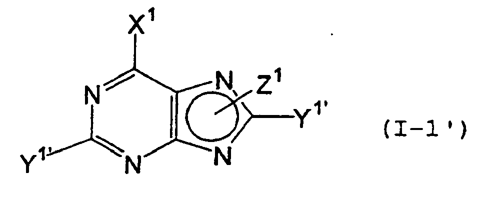
   wherein
   X¹ and Z¹ are as defined above, and
   one of Y^{1'} is a group represented by the formula: Me₂(OH)C-C≡C-, and the other Y^{1'} is a hydrogen atom, or a salt thereof, with a base (2).
[9] A production method of a compound represented by the formula (I-1"'): 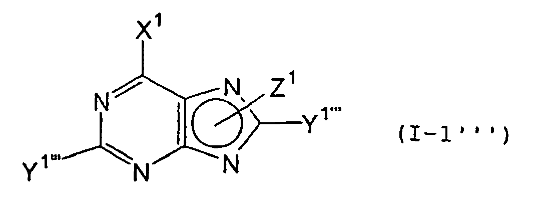 wherein
   X¹ is an alkyl group, an alkoxy group, an aryl group, an optionally protected amino group, a halogen atom or a hydrogen atom,
   Z¹ is an alkyl group, an amino-protecting group or a hydrogen atom, which is attached to a nitrogen atom at the 7- or 9-position of the purine nucleus, and
   one of Y^{1"'} is a group represented by the formula: A-C≡C-,
   wherein A is an aryl group optionally having substituents or a heterocyclic group optionally having substituents, and the other Y^{1"'} is a hydrogen atom,
   or a salt thereof, which comprises reacting a compound represented by the formula (I-1"): 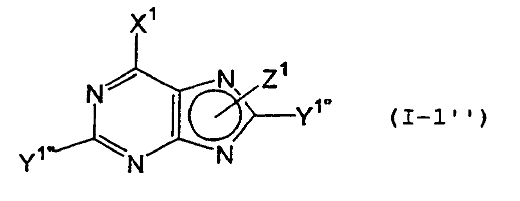 wherein
   X¹ and Z¹ are as defined above, and
   one of Y^{1"} is a group represented by the formula: HC≡C-, and the other Y^{1"} is a hydrogen atom,
   or a salt thereof, with a compound represented by the formula (IV): A-X wherein A is as defined above, and X is a halogen atom, in the presence of a metal catalyst and a base (1).
[10] A production method of a compound represented by the formula (I-1"') : 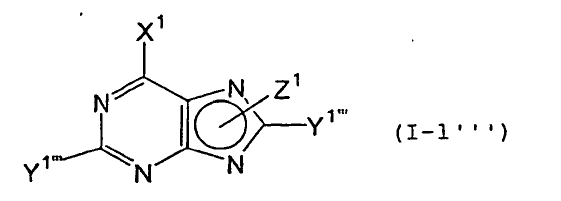 wherein
   X¹ is an alkyl group, an alkoxy group, an aryl group, an optionally protected amino group, a halogen atom or a hydrogen atom,
   one of Y^{1"'} is a group..represented by the formula: A-C≡C-, wherein A is an aryl group optionally having substituents or a heterocyclic group optionally having substituents, and the other Y^{1'"} is a hydrogen atom, and
   Z¹ is an alkyl group, an amino-protecting group or a hydrogen atom, which is attached to a nitrogen atom at the 7- or 9-position of the purine nucleus,
   or a salt thereof, which comprises the following steps (a)-(c) :
      (a) a step of obtaining a compound represented by the formula (I-1') : 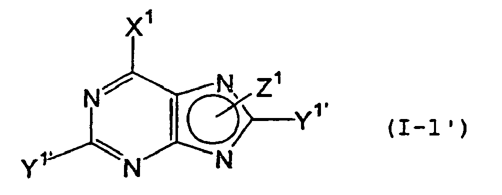 wherein
         X¹ is an alkyl group, an alkoxy group, an aryl group, an optionally protected amino group, a halogen atom or a hydrogen atom,
         Z¹ is an alkyl group, an amino-protecting group or a hydrogen atom, which is attached to a nitrogen atom at the 7- or 9-position of the purine nucleus, and
         one of Y^{1'} is a group represented by the formula: Me₂(OH)C-C≡C-, and the other Y^{1'} is a hydrogen atom,
         or a salt thereof, which comprises reacting a compound
         represented by the formula (II-1): 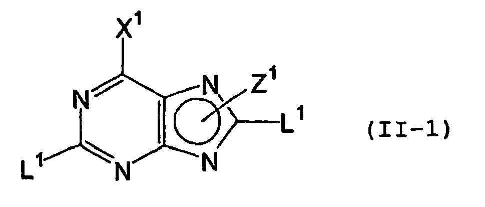 wherein
         X¹ and Z¹ are as defined above, and
         one of L¹ is a halogen atom, and the other L¹ is a hydrogen atom, provided that when X¹ is a halogen atom, L¹ is a halogen atom having higher leaving ability than the halogen atom represented by X¹,
         or a salt thereof, with a compound represented by the formula (III): Me₂(OH)C-C≡CH, in the presence of a metal catalyst and a base (1),
      (b) a step of obtaining a compound represented by the formula (I-1"): 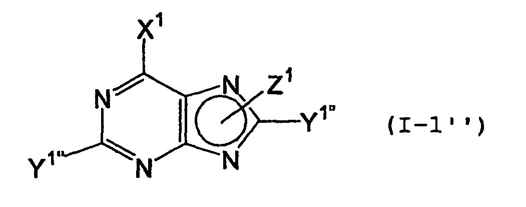 wherein
         X¹ and Z¹ are as defined above, and
         one of Y^{1"} is a group represented by the formula: HC≡C-, and the other Y^{1"} is a hydrogen atom, or a salt thereof, which comprises treating a compound of the formula (I-1') obtained in the step (a) or a salt thereof with a base (2), and
      (c) a step of reacting a compound of the formula (I-1") obtained in the step (b) or a salt thereof, with a compound represented by the formula (IV): A-X wherein A is an aryl group optionally having substituents or a heterocyclic group optionally having substituents, and X is a halogen atom, in the presence of a metal catalyst and a base (1).
[11] A production method of a compound represented by the formula (I-2'): 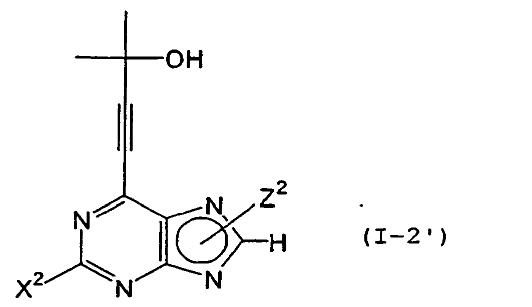 wherein
   X² is an alkyl group, an alkoxy group, an aryl group, an optionally protected amino group, a halogen atom or a hydrogen atom, and
   Z² is an alkyl group, an amino-protecting group or a hydrogen atom, which is attached to a nitrogen atom at the 7- or 9-position of the purine nucleus,
   or a salt thereof, which comprises reacting a compound represented by the formula (II-2): 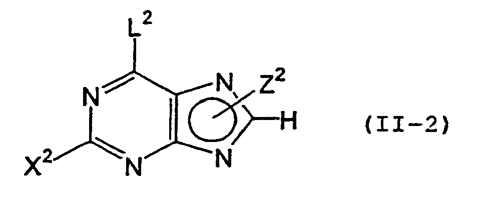
   wherein
   X² and Z² are as defined above, and
   L² is a halogen atom, provided that when X² is a halogen atom,
   L² is a halogen atom having higher leaving ability than the halogen atom represented by X², or the same halogen atom as X², or a salt thereof, with a compound represented by the formula (III): Me₂(OH)C-C≡CH, in the presence of a metal catalyst and a base (1).
[12] A production method of a compound represented by the formula (I-2"): 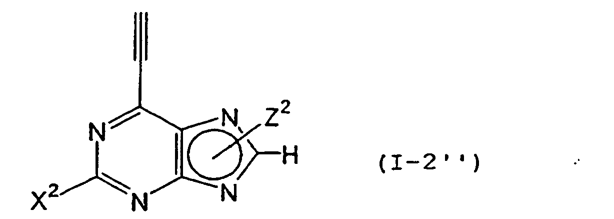 wherein
   X² is an alkyl group, an alkoxy group, an aryl group, an optionally protected amino group, a halogen atom or a hydrogen atom, and
   z² is an alkyl group, an amino-protecting group or a hydrogen atom, which is attached to a nitrogen atom at the 7- or 9-position of the purine nucleus,
   or a salt thereof, which comprises treating a compound represented by the formula (I-2'): 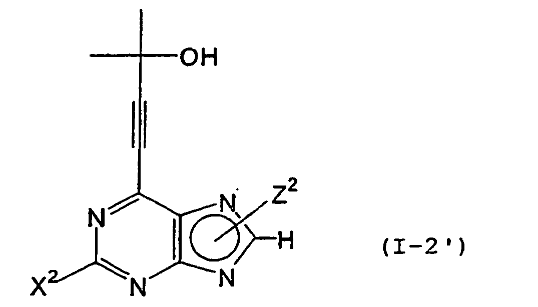
   wherein
   X² and Z² are as defined above,
   or a salt thereof, with a base (2).
[13] A production method of a compound represented by the formula (I-2"'): 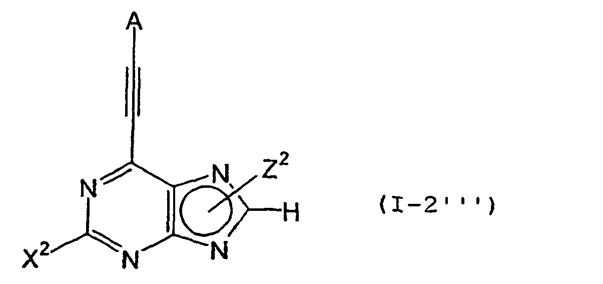 wherein
   A is an aryl group optionally having substituents or a heterocyclic group optionally having substituents,
   X² is an alkyl group, an alkoxy group, an aryl group, an optionally protected amino group, a halogen atom or a hydrogen atom, and
   Z² is an alkyl group, an amino-protecting group
   or a hydrogen atom, which is attached to a nitrogen atom at the 7- or 9-position of the purine nucleus,
   or a salt thereof, which comprises reacting a compound represented by the formula (I-2"): 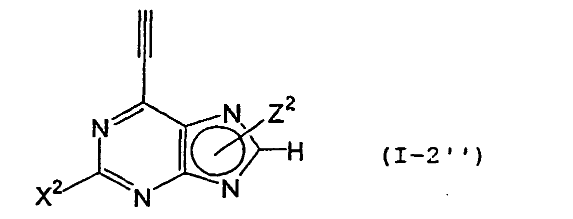
   wherein
   X² and Z² are as defined above,
   or a salt thereof, with a compound represented by the formula (IV): A-X, wherein A is as defined above, and X is a halogen atom, in the presence of a metal catalyst and a base (1).
[14] A production method of a compound represented by the formula (I-2"') : 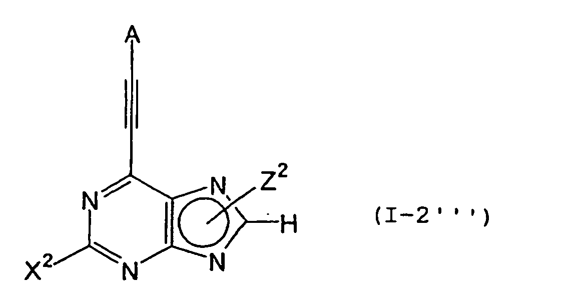 wherein
   A is an aryl group optionally having substituents or a heterocyclic group optionally having substituents,
   X² is an alkyl group, an alkoxy group, an aryl group, an optionally protected amino group, a halogen atom or a hydrogen atom, and
   Z² is an alkyl group, an amino-protecting group or a hydrogen atom, which is attached to a nitrogen atom at the 7- or 9-position of the purine nucleus,
   or a salt thereof, which comprises the following steps (a)-(c) :
      (a) a step of obtaining a compound represented by the formula (I-2'): 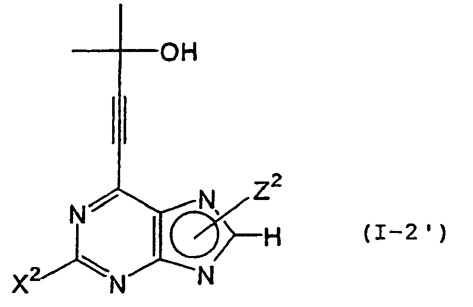 wherein
         X² is an alkyl group, an alkoxy group, an aryl group, an optionally protected amino group, a halogen atom or a hydrogen atom, and
         Z² is an alkyl group, an amino-protecting group or a hydrogen atom, which is attached to a nitrogen atom at the 7- or 9-position of the purine nucleus,
         or a salt thereof, which comprises reacting a compound represented by the formula (II-2) : 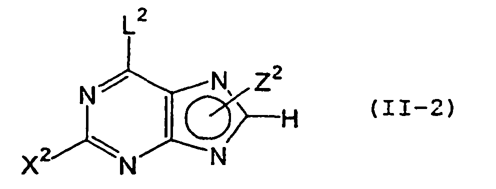
         wherein
         X² and Z² are as defined above, and
         L² is a halogen atom, provided that when X² is a halogen atom,
         L² is a halogen atom having higher leaving ability than the halogen atom represented by X², or the same halogen atom as X², or a salt thereof, with a compound represented by the formula (III): Me₂(OH)C-C≡CH, in the presence of a metal catalyst and a base (1),
      (b) a step of obtaining a compound represented by the formula (I-2") : 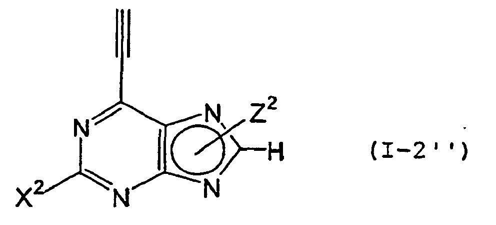 wherein
         X² and Z² are as defined above,
         or a salt thereof, which comprises treating a compound of the formula (I-2') obtained in the step (a) or a salt thereof, with a base (2), and
      (c) a step of reacting a compound of the formula (I-2") obtained in the step (b) or a salt thereof, with a compound represented by the formula (IV): A-X, wherein A is an aryl group optionally having substituents or a heterocyclic group optionally having substituents, and X is a halogen atom, in the presence of a metal catalyst and a base (1).
[15] The production method of any of the above-mentioned [9], [10], [11], [13] and [14], wherein the metal catalyst is a palladium compound.
[16] The production method of any of the above-mentioned [9], [10], [11], [13] and [14], wherein the metal catalyst is a combination of a palladium compound and a copper compound.
[17] The production method of the above-mentioned [15] or [16] wherein the palladium compound is bis(triphenylphosphine)palladium dichloride or tetrakis(triphenylphosphine)palladium.
[18] The production method of the above-mentioned [16], wherein the copper compound is at least one selected from cuprous iodide, cuprous bromide and cuprous chloride.
[19] The production method of any of the above-mentioned [9], [10], [11], [13] and [14]-[18], wherein the base (1) is an amine compound.
[20] The production method of the above-mentioned [19], wherein the amine compound is trialkylamine.
[21] The production method of the above-mentioned [20],
   wherein the trialkylamine is triethylamine or ethyldiisopropylamine.
[22] The production method of any of the above-mentioned [8], [10], [12], and [14]-[21], wherein the base (2) is alkali metal hydroxide or alkali metal carbonate.

### Best Mode for Embodying the Invention

The present invention is explained in detail in the following.

The present invention relates to an alkynylpurine compound represented by the following formula (I) wherein an alkynyl moiety is attached to any of the 2-, 6- and 8-positions of the purine nucleus shown with arrows (→), which is useful as an intermediate for the production of medicaments, (this compound encompasses isomers such as tautomer and the like, and optionally further has substituents to be described in detail in the following), a salt thereof, and a production method thereof.

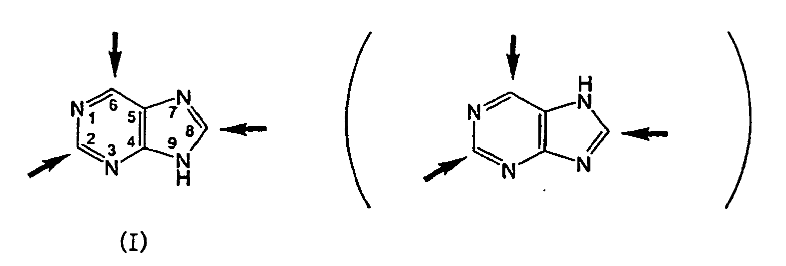

More specifically, this invention relates to a production method of an alkynylpurine compound represented by the formula (I-1) and the formula (I-2) (hereinafter to be referred to as compound (I-1) and compound (I-2)) and a salt thereof.

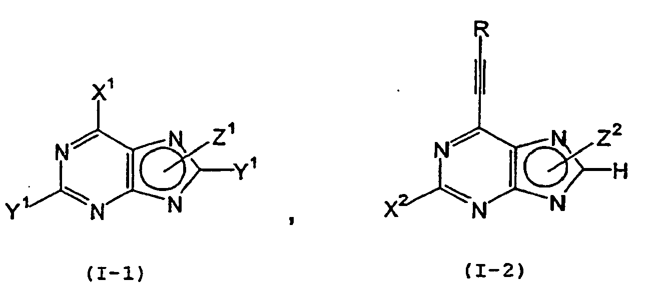

In the above-mentioned compound (I-1), X¹ is an alkyl group, an alkoxy group, an aryl group, an optionally protected amino group, a halogen atom or a hydrogen atom, one of Y¹ has, as an alkynyl moiety, a group represented by the formula: R-C≡C-, wherein R is a hydrogen atom, the other Y¹ is a hydrogen atom, and Z¹ is an alkyl group, an amino-protecting group or a hydrogen atom, which is attached to a nitrogen atom at the 7-or 9-position of the purine nucleus. In the above-mentioned compound (I-2), as an alkynyl moiety, a group represented by the formula: R-C≡C-, wherein R is a hydrogen atom or a hydrocarbon group optionally having substituents, is attached to the 6-position of the purine nucleus, X² is an alkyl group, an alkoxy group, an aryl group, an optionally protected amino group or a halogen atom, Z² is an alkyl group, an amino-protecting group or a hydrogen atom, which is attached to a nitrogen atom at the 7- or 9-position of the purine nucleus.

### Definition

In the present invention, "R" is a hydrogen atom or a hydrocarbon group optionally having substituents.

As the "hydrocarbon group" of the "hydrocarbon group optionally having substituents" denoted by R, for example, C₁₋₁₀ alkyl (e.g., methyl, ethyl, n-propyl, isopropyl, n-butyl, isobutyl, tert-butyl, pentyl, hexyl, heptyl, octyl, nonyl, decyl etc.) and the like can be mentioned. Of these, C₁₋₆ alkyl is preferable.

As the "substituent" of the above-mentioned "hydrocarbon group optionally having substituents", C₁₋₆ alkyl (e.g., methyl, ethyl, n-propyl, isopropyl, n-butyl, isobutyl, tert-butyl etc.), hydroxy and the like can be mentioned. The kind, position and number of the substituents are not particularly limited.

As the above-mentioned "hydrocarbon group optionally having substituents", C₁₋₆ alkyl optionally having substituents is preferable. Of those exemplified, C₁₋₆ alkyl having hydroxy at the 1-position as a substituent is preferable. For example, 1-methyl-1-hydroxyethyl (i.e., Me₂(OH)C-) and the like are mentioned.

As R, preferred are hydrogen atom and C₁₋₆ alkyl optionally having substituents.

More specifically, hydrogen atom and C₁₋₆ alkyl having hydroxy at the 1-position as a substituent (e.g., 1-methyl-1-hydroxyethyl) are particularly preferable as R.

The reason for particular preference of hydrogen atom as R is that, when R is a hydrogen atom, the alkynyl moiety represented by R-C≡C- (i.e., ethynyl group) is still more easily modified due to its high reactivity [see Step C described in detail in the following].

The reason for particular preference of the "C₁₋₆ alkyl having hydroxy at the 1-position" as R is that, when R is -C₁₋₆ alkyl having hydroxy at the 1-position", the alkynyl moiety represented by R-C≡C- can be easily converted to an ethynyl group via β-elimination by a treatment with a base [see the treatment with base (2) in Step B explained in the following].

In the present invention, "X¹" is an alkyl group, an alkoxy group, an aryl group, an optionally protected amino group, a halogen atom or a hydrogen atom, an "X²" is an alkyl group, an alkoxy group, an aryl group, an optionally protected amino group or a halogen atom.

As the "alkyl group" denoted by "X¹" and "X²", for example, C₁₋₁₀ alkyl (e.g., methyl, ethyl, n-propyl, isopropyl, n-butyl, isobutyl, tert-butyl, pentyl, hexyl, heptyl, octyl, nonyl, decyl etc.) and the like are mentioned for both.

As the "alkoxy group" denoted by "X¹" and "X²", for example, C₁₋₆ alkoxy (e.g., methoxy, ethoxy, n-propoxy, isopropoxy, n-butoxy, tert-butoxy etc.) and the like are mentioned for both.

As the "aryl group" denoted by "X¹" and "X²", for example, C₆₋₁₂ aryl (e.g., phenyl, biphenylyl, naphthyl etc.) and the like are mentioned for both.

The "protecting group" of the "optionally protected amino group" denoted by "X¹" and "X²" is not particularly limited as long as it is an amino-protecting group known to those of ordinary skill in the art of the organic chemistry, and, for example, a hetero ring group (e.g., a 5 to 8-membered hetero ring group containing, besides carbon atoms, 1 to 3 hetero atoms selected from the group consisting of nitrogen atom, oxygen atom and sulfur atom, such as tetrahydropyranyl (e.g., tetrahydropyran-2-yl etc.) and the like), C₇₋₁₅ aralkyl (e.g., benzyl etc.), C₁₋₅ acyl (e.g., acetyl etc.), trialkylsilyl (e.g., trimethylsilyl etc.), carbamate (e.g., tert-butylcarbamate) and the like are mentioned.

The "amino group" of the "optionally protected amino group" denoted by "X¹" and "X²" is optionally substituted by 1 or 2 substituents. As the substituent, for example, C₁₋₆ alkyl (e.g., methyl, ethyl, n-propyl, isopropyl, n-butyl, isobutyl, tert-butyl, pentyl, hexyl etc.), C₆₋₁₂ aryl (e.g., phenyl, naphthyl etc.) and the like are mentioned. When the amino group has two substituents, the substituents may be the same or different.

As the "halogen atom" denoted by "X¹" and "X²", for example, fluorine atom, chlorine atom, bromine atom, iodine atom and the like are mentioned, particularly preferably chlorine atom.

In a compound represented by the formula (I-1) in the present specification, one of "Y¹" is a group represented by the formula: R-C≡C- wherein R is as defined above), and the other "Y¹" is a hydrogen atom.

In a compound represented by the formula (I-1') in the present specification, one of "Y^{1'}" is a group represented by the formula: Me₂(OH)C-C≡C-, and the other "Y^{1'}" is a hydrogen atom.

In a compound represented by the formula (I-1") in the present specification, one of "Y^{1"}" is a group represented by the formula: HC≡C-, and the other "Y^{1"}" is a hydrogen atom.

In a compound represented by the formula (I-1"') in the present specification, one of "Y^{1"'}" is a group represented by the formula: A-C≡C- wherein A is as defined in the following "A-X"), and the other "Y^{1"'}" is a hydrogen atom.

The "Z¹" and "Z²" are each an alkyl group, an amino-protecting group or a hydrogen atom, which is attached to a nitrogen atom at the 7- or 9-position of the purine nucleus.

As the "alkyl group" denoted by "Z¹" and "Z²", for example, C₁₋₆ alkyl (e.g., methyl, ethyl, n-propyl, isopropyl, n-butyl, isobutyl, tert-butyl etc.) and the like are mentioned for both.

As the "amino-protecting group" denoted by "Z¹" and "Z²", amino-protecting groups well known to those of ordinary skill in the art of the organic synthesis can be used. As the amino-protecting group, for example, hetero ring group (e.g., 5 to 8-membered hetero ring group containing, besides carbon atoms, 1 to 3 hetero atoms selected from the group consisting of nitrogen atom, oxygen atom and sulfur atom, such as tetrahydropyranyl (e.g., tetrahydropyran-2-yl etc.) and the like), C₇₋₁₅ aralkyl (e.g., benzyl etc.), C₁₋₅ acyl (e.g. , acetyl etc.), trialkylsilyl (e.g., trimethylsilyl etc.), carbamate (e.g., tert-butylcarbamate) and the like are mentioned.

The "Z¹" and "Z²" are each preferably a hydrogen atom and an amino-protecting group, and as the amino-protecting group, a hetero ring group is preferable, and tetrahydropyranyl (e.g., tetrahydropyran-2-yl etc.) is particularly preferable. As the amino-protecting group, C₇₋₁₅ aralkyl is also preferable, and benzyl is particularly preferable.

In a compound represented by the formula (II-1) in the present specification, one of "L¹" is a halogen atom (e.g., fluorine atom, chlorine atom, bromine atom, iodine atom etc.), and the other "L¹" is a hydrogen atom. However, when X¹ is a halogen atom, one of L¹ is a "halogen atom having higher leaving ability than the halogen atom represented by X¹".

In a compound represented by the formula (II-2) in the present specification, "L²" is a halogen atom (e.g., fluorine atom, chlorine atom, bromine atom, iodine atom etc.). However, when X² is a halogen atom, L² is a "halogen atom having higher leaving ability than the halogen atom represented by X²", or the "same halogen atom as X²". When X² is a halogen atom in a - compound represented by the formula (II-2), L² may be the "same halogen atom as X²" because, when the same halogen atoms are present at the 2- and 6-positions of the purine nucleus, the halogen atom at the 6-position generally has higher leaving ability than the halogen atom at the 2-position.

The above-mentioned "halogen atom having higher leaving ability than the halogen atom represented by X¹" and "halogen atom having higher leaving ability than the halogen atom represented by X²" each mean a halogen atom having higher leaving ability than the halogen atom represented by X¹ or X², according to the leaving ability of halogen atom: F<Cl<Br<I. For example, when X¹ and X² are each a fluorine atom, L¹ and L² are each selected from the group consisting of chlorine atom, bromine atom and iodine atom, when X¹ and X² are each a chlorine atom, L¹ and L² are each a bromine atom or an iodine atom, when X¹ and X² are each a bromine atom and L¹ and L² are each an iodine atom.

When X¹ and X² are each a halogen atom, it is particularly preferable that X¹ and X² be each a chlorine atom and L¹ and L² be each an iodine atom, in view of the difference in leaving ability and easiness of modification thereafter.

When X¹ and X² are each other than a halogen atom, L¹ and L² are not particularly limited, and a halogen atom generally having higher leaving ability, particularly an iodine atom, is preferable.

In the compound represented by "A-X" in the present invention [i.e. a compound represented by the formula (IV)], "A" is an aryl group optionally having substituents or a heterocyclic group optionally having substituents and "X" is a halogen atom.

As the "aryl group" of the "aryl group optionally having substituents" denoted by "A", for example, C₆₋₁₂ aryl (e.g., phenyl, biphenylyl, naphthyl etc.) and the like are mentioned, with preference given to phenyl.

As the "substituent" of the above-mentioned "aryl group optionally having substituents", for example, halogen atoms (e.g., fluorine atom, chlorine atom, bromine atom, iodine atom etc.), C₁₋₆ alkyl (e.g., methyl, ethyl, n-propyl, isopropyl, n-butyl, isobutyl, tert-butyl, pentyl, hexyl etc.), C₁₋₆ alkoxy (e.g., methoxy, ethoxy, n-propoxy, isopropoxy, n-butoxy, tert-butoxy etc.), nitro and the like are mentioned. The kind, position and number of the substituents are not particularly limited.

As the above-mentioned "aryl group optionally having substituents", for example, phenyl; halophenyl such as chlorophenyl (e.g., 2- or 4-chlorophenyl etc.), fluorophenyl (e.g., 2- or 4-fluorophenyl etc.), bromophenyl (e.g., 2- or 4-bromophenyl etc.) and the like; C₁₋₆ alkylphenyl (e.g., 3- or 4-methylphenyl etc.); C₁₋₆ alkoxyphenyl (e.g., 4-methoxyphenyl etc.); nitrophenyl (e.g., 4-nitrophenyl etc.) and the like are preferable.

As the "heterocyclic group" of the "heterocyclic group optionally having substituents" denoted by "A", for example, 5 to 8-membered heterocyclic groups containing, besides carbon atoms, 1 to 3 hetero atoms selected from the group consisting of nitrogen, oxygen and sulfur atom are mentioned. As "A", nitrogen-containing heterocyclic groups such as pyridyl (e.g., pyridin-3-yl etc.), pyrimidyl (e.g., pyrimidin-2-yl, pyrimidin-3-yl, pyrimidin-5-yl etc.) and the like are particularly preferable.

As the "substituent" of the above-mentioned "heterocyclic group optionally having substituents", for example, halogen atoms (e.g., fluorine atom, chlorine atom, bromine atom, iodine atom etc.), oxo and the like are mentioned. The kind, position and number of the substituents are not particularly limited.

As the above-mentioned "heterocyclic group optionally having substituents", for example, pyridin-3-yl, 2,4-dioxopyrimidin-5-yl and the like are preferable.

As the "halogen atom" denoted by "X", for example, fluorine atom, chlorine atom, bromine atom, iodine atom and the like are mentioned, with preference given to bromine atom and iodine atom from the aspect of reactivity.

As the compound represented by the formula (IV): A-X, dihalobenzene (e.g., 1-bromo-4-chlorobenzene, 2-chloro-4-iodobenzene, 1-chloro-2-iodobenzene, 1-fluoro-2-iodobenzene etc.), C₁₋₆ alkylhalobenzene (e.g., 4-bromotoluene, 4-iodotoluene, 3-bromotoluene, 3-iodotoluene etc.), C₁₋₆ alkoxyhalobenzene (e.g., 4-bromoanisole, 4-iodoanisole etc.), nitrohalobenzene (e.g., 1-bromo-4-nitrobenzene, 1-iodo-4-nitrobenzene etc.), halopyridine (e.g., 3-bromopyridine, 3-iodopyridine etc.) and dioxohalopyrimidine (e.g., 2,4-dioxo-5-iodopyrimidine etc.) are particularly preferable.

As the "metal catalyst" to be used in the present invention, a palladium compound and a combination of a palladium compound and a copper compound are mentioned.

As the "palladium compound" to be used in the present invention, bis(triphenylphosphine)palladium dichloride [i.e. (Ph₃P)₂PdCl₂], tetrakis (triphenylphosphine) palladium [i.e. (Ph₃P)₄Pd], palladium acetate [i.e. Pd(OAc)₂], bis (acetylacetonato) palladium [i.e. Pd(acac)₂], [1,1'-bis (diphenylphosphino) ferrocene]palladium(II) chloride [i.e. PdCl₂(dppf)₂] and the like are mentioned. Of these, bis(triphenylphosphine)palladium dichloride and tetrakis(triphenylphosphine)palladium are preferable, from the aspect of solubility in a solvent.

When a combination of a palladium compound and a copper compound is used as a metal catalyst, the palladium compound includes by the aforementioned palladium catalysts and, from the aspect of solubility in a solvent, bis(triphenylphosphine)palladium dichloride is particularly preferable. When a combination of a palladium compound and a copper compound is used as a metal catalyst, as the "copper compound", for example, cuprous halide such as cuprous iodide, cuprous bromide, cuprous chloride and the like and cuprous cyanide are mentioned. Of these, cuprous halide is preferable, and cuprous iodide is particularly preferable in view of the catalytic ability. In the present invention, two or more kinds of copper compounds may be used.

When a palladium compound and a copper compound are used in combination as a metal catalyst, the kind of the palladium compound and copper compound to be used is not particularly limited, and from the above-mentioned aspects, a combination of bis(triphenylphosphine)palladium dichloride and cuprous iodide is particularly preferable.

As the "base (1)" to be used in the present invention, for example, amine compound, alkali metal hydroxide, alkali metal carbonate, basic heterocyclic compound and the like are mentioned.

As the "amine compound", for example, trialkylamine and the like are mentioned, wherein the alkyl moiety of the trialkylamine is, for example, C₁₋₆ alkyl (e.g., methyl, ethyl, n-propyl, isopropyl, n-butyl etc.), and the alkyl groups may be the same or different. As trialkylamine, for example, triethylamine, ethyldiisopropylamine and the like are mentioned.

As the "alkali metal hydroxide", for example, sodium hydroxide, potassium hydroxide and the like are mentioned.

As the "alkali metal carbonate", for example, sodium carbonate, potassium carbonate and the like are mentioned.

As the "basic heterocyclic compound", for example, pyridine and the like are mentioned.

As the "base (1)" to be used in the present invention, amine compound is preferable. As the amine compound, trialkylamine is preferable, and particularly preferred therefrom are triethylamine and ethyldiisopropylamine.

As the "base (2)" to be used in the present invention, for example, alkali metal hydroxide, alkali metal carbonate, amine and the like are mentioned.

As the "alkali metal hydroxide", for example, sodium hydroxide, potassium hydroxide and the like are mentioned.

As the "alkali metal carbonate", for example, sodium carbonate, potassium carbonate, sodium hydrogencarbonate and the like are mentioned.

As the "amine", for example, triethylamine and the like are mentioned.

As the "base (2)" to be used in the present invention, alkali metal hydroxide and alkali metal carbonate are preferable. Of these, alkali metal hydroxide is preferable, and sodium hydroxide and potassium hydroxide are particularly preferable, in view of the solubility in water.

The salts of the compound according to the present invention are not particularly limited, and, for example, a salt of the compound according to the present invention with an organic acid (e.g., methanesulfonic acid, trifluoromethanesulfonic acid, p-toluenesulfonic acid etc.), a salt of the compound according to the present invention with an inorganic acid (e.g., hydrochloric acid, sulfuric acid, phosphoric acid, nitric acid etc.), and ammonium salt of the compound according to the present invention (e.g., tetraalkylammonium salt etc.) and the like are mentioned.

### Production method of alkynylpurine compound

The production method of the alkynylpurine compound according to the present invention is explained in the following.

First, the production methods of the alkynylpurine compound represented by the above-mentioned formula (I-1) are explained by referring to the following Scheme 1. 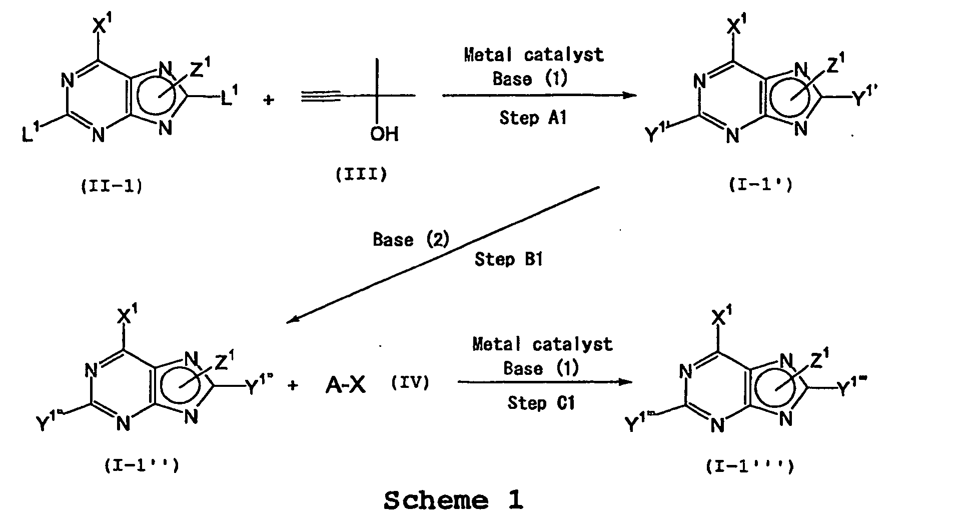 In the Scheme, each symbol is as defined above. 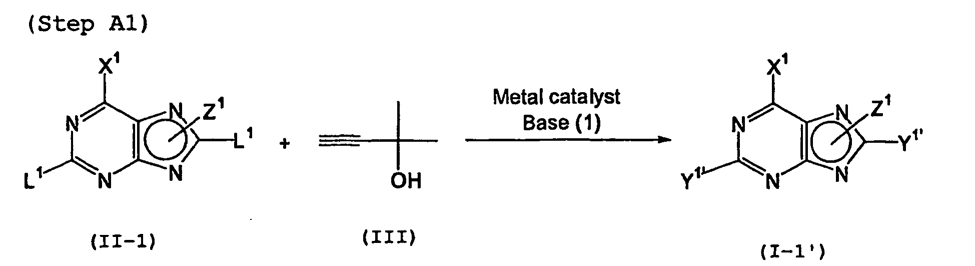 wherein each symbol is as defined above.

In Step A1, a compound represented by the formula (I-1') wherein one of Y^{1'} is Me₂(OH)C-C≡C- and the other Y^{1'} is a hydrogen atom (hereinafter sometimes to be referred to as compound (I-1')) or a salt thereof is obtained by reacting a compound represented by the formula (II-1) wherein one of L¹ is a halogen atom and the other L¹ is a hydrogen atom (hereinafter sometimes to be referred to as compound (II-1)) or a salt thereof, with a compound represented by the formula (III) (hereinafter sometimes to be referred to as compound (III)), in the presence of a metal catalyst and a base (1).

In Step A1, a palladium compound may be used alone as a metal catalyst, as defined above. When a palladium compound is used alone, the palladium compound is particularly preferably bis(triphenylphosphine)palladium dichloride. The amount of the palladium compound to be used is not particularly limited as long as a catalytic amount relative to compound (II-1) or a salt thereof is used. It is generally 0.01-1.0 mmole, preferably 0.05-0.2 mmole, per 1 mmole of compound (II-1) or a salt thereof. As the metal catalyst, a combination of a palladium compound and a copper compound is preferable, in view of the activity of the catalyst. When the palladium compound and the copper compound are to be used in combination, bis(triphenylphosphine)palladium dichloride is particularly preferable as the palladium compound, and cuprous iodide is particularly preferable as the copper compound. The amount of the palladium compound to be used and the amount of the copper compound to be used are not particularly limited as long as a catalytic amount relative to compound (II-1) or a salt thereof is used. The amount of the palladium compound to be used is generally 0.01-1.0 mmole, preferably 0.05-0.2 mmole, per 1 mmole of compound (II-1) or a salt thereof, and the amount of the copper compound to be used is generally 0.01-1.0 mmole, preferably 0.05-0.2 mmole, per 1 mmole of compound (II-1) or a salt thereof.

In Step A1, an amine compound is preferably used as the base (1), as defined above. In addition, trialkylamine is preferably used as an amine compound. As the trialkylamine, triethylamine and ethyldiisopropylamine are preferable. The amount of the base (1) to be used is generally 1.0-5.0 mmole, preferably 1.0-1.5 mmole, per 1 mmole of compound (II-1) or a salt thereof.

The compound (II-1) or a salt thereof to be used in Step A1 can be synthesized according to the method described in, for example, US 4,719,295 to Cook et al., and a method analogous thereto.

The compound (III) to be used in Step A1 is commercially available.

The salt of compound (II-1) to be used in Step A1 is not particularly limited, and, for example, a salt of compound (II-1) with an organic acid (e.g., methanesulfonic acid, trifluoromethanesulfonic acid, p-toluenesulfonic acid etc.), a salt of compound (II-1) with an inorganic acid (e.g., hydrochloric acid, sulfuric acid, phosphoric acid, nitric acid etc.), and ammonium salt thereof (e.g., tetraalkylammonium salt etc.) and the like are mentioned.

In Step A1, the amount of compound (III) to be used is generally 1.0-5.0 mmole, preferably 1.0-1.5 mmole, per 1 mmole of compound (II-1) or a salt thereof.

In Step A1, the solvent is not particularly limited as long as the reaction is not adversely affected by the solvent, and, for example, aprotic solvents such as DMF (N,N-dimethylformamide), DMSO (dimethyl sulfoxide), N,N-dimethylacetamide, THF (tetrahydrofuran) and the like, and the like are mentioned. Of these, DMF is preferable. The amount of the solvent to be used is not particularly limited and it is generally 1.5-100 mL, preferably 1.5-50 mL, per 1 mmole of compound (II-1) or a salt thereof.

In Step A1, the reaction time varies depending on the reagents and the solvent to be used, and is generally 1-5 hrs.

In Step A1, the reaction temperature varies depending on the reagents and the solvent to be used, and is generally 40°C-150°C, preferably 80°C-100°C.

As the salt of compound (I-1'), those similar to the salts recited above are mentioned. 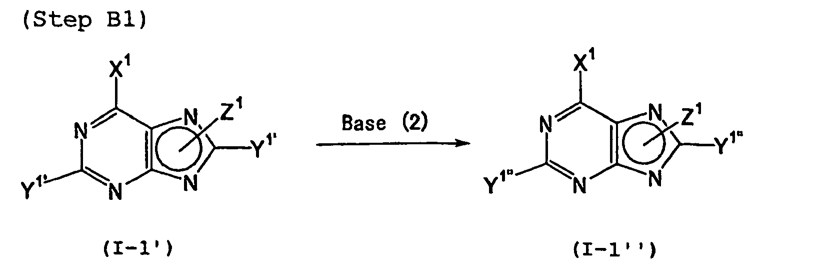 wherein each symbol is as defined above.

In Step B1, a compound represented by the formula (I-1") wherein one of Y^{1"} is HC≡C- and the other Y^{1"} is a hydrogen atom (hereinafter sometimes to be referred to as compound (I-1")) or a salt thereof is obtained by treating compound (I-1') obtained in the above-mentioned Step A1, wherein one of Y^{1'} is Me₂(OH)C-C≡C-, and the other Y^{1'} is a hydrogen atom, or a salt thereof with a base (2).

In Step B1, alkali metal hydroxide is particularly preferably used as the base (2), as defined above. Particularly, sodium hydroxide and potassium hydroxide are preferable.

In Step B1, the amount of the base (2) to be used is generally 1-20 mmole, preferably 3-10 mmole, per 1 mmole of compound (I-1') or a salt thereof.

In Step B1, the solvent is not particularly limited as long as the reaction is not adversely affected by the solvent, and, for example, aprotic solvents such as toluene, xylene, THF, MTBE (tert-butylmethyl ether) and the like, and the like are mentioned, with particular preference given to toluene. The amount of the solvent to be used is not particularly limited, and is generally 10-500 mL, preferably 50-200 mL, per 1 mmole of compound (I-1') or a salt thereof.

In Step B1, the reaction time varies depending on the reagents and the solvent to be used, and is generally 1-5 hrs.

In Step B1, the reaction temperature varies depending on the substrate as well as the reagents and the solvent to be used, and is generally 30°C-150°C, preferably 60°C-110°C. Particularly when compound (I-1') has Me₂(OH)C-C≡C- at the 8-position, the reaction temperature is preferably 60°C-80°C, because the reaction rate becomes higher.

As the salt of compound (I-1"), those similar to the salts recited above are mentioned. 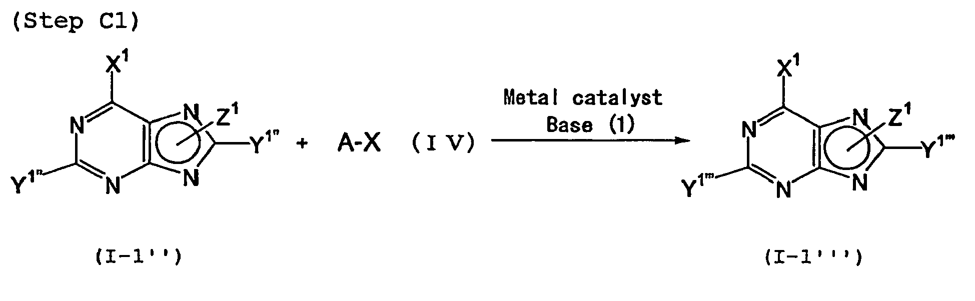 wherein each symbol is as defined above.

In Step C1, a compound represented by the formula (I-1"') wherein one of Y^{1"'} is A-C≡C- and the other Y^{1"'} is a hydrogen atom (hereinafter sometimes to be referred to as compound (I-1"')) or a salt thereof is obtained by reacting, in the presence of a metal catalyst and a base (1), compound (I-1") obtained in Step B1, wherein one of Y^{1"} is HC≡C- and the other Y^{1"} is a hydrogen atom, or a salt thereof with a halide compound represented by the formula (IV) (hereinafter sometimes to be referred to as compound (IV)) .

In Step C1, a palladium compound may be used alone as a metal catalyst, as defined above. When a palladium compound is used alone, a palladium compound is particularly preferably bis(triphenylphosphine)palladium dichloride. The amount of the palladium compound to be used is not particularly limited as long as a catalytic amount relative to compound (I-1") or a salt thereof is used, and is generally 0.01-1.0 mmole, preferably 0.05-0.2 mmole, per 1 mmole of compound (I-1") or a salt thereof. As a metal catalyst, a combination of a palladium compound and a copper compound is preferable. When a palladium compound and a copper compound are used in combination, bis(triphenylphosphine)palladium dichloride is a particularly preferable palladium compound, and cuprous iodide is a particularly preferable copper compound. The amount of the palladium compound to be used and the amount of the copper compound to be used are not particularly limited as long as a catalytic amount is used relative to compound (I-1") or a salt thereof. The amount of the palladium compound to be used is generally 0.01-1.0 mmole, preferably 0.05-0.2 mmole, per 1 mmole of compound (I-1") or a salt thereof, and the amount of the copper compound to be used is generally 0.01-1.0 mmole, preferably 0.05-0.2 mmole, per 1 mmole of compound (I-1") or a salt thereof.

In Step C1, an amine compound is preferably used as a base (1), as defined above. As the amine compound, trialkylamine is preferable. As the trialkylamine, triethylamine and ethyldiisopropylamine are preferable. The amount of the base (1) to be used is generally 1.0-5.0 mmole, preferably 1.0-3.0 mmole, per 1 mmole of compound (I-1") or a salt thereof.

In Step C1, the amount of compound (IV) to be used is generally 1.0-2.0 mmole, preferably 1.0-1.3 mmole, per 1 mmole of compound (I-1") or a salt thereof.

In Step C1, the solvent is not particularly limited as long as the reaction is not adversely affected by the solvent, and, for example, aprotic solvents such as DMF, N,N-dimethylacetamide, DMSO, THF and the like, and the like are mentioned, with preference given to DMF. The amount of the solvent to be used is not particularly limited, and is generally 1-200 mL, preferably 5-20 mL, per 1 mmole of compound (I-1") or a salt thereof.

In Step C1, the reaction time varies depending on the reagents and solvent to be used, and is generally 2-8 hrs.

In Step C1, the reaction temperature varies depending on the reagents and the solvent to be used, and is generally 50°C-150°C, preferably 80°C-100°C.

As the salt of compound (I-1"'), those similar to the salts recited above are mentioned.

Accordingly, alkynylpurine compound represented by the formula (I-1) is considered to encompass compounds (I-1'), (I-1"), (I-1"') (including isomers such as tautomer and the like, obtainable in each step) and salts thereof obtained by the reactions described in Scheme 1, as well as compounds and salts thereof obtained by the methods according to Scheme 1.

As with the production methods of the alkynylpurine compound represented by the formula (I-1), the production methods of the alkynylpurine compound represented by the above-mentioned formula (I-2) are explained in the following while referring to the following Scheme 2. 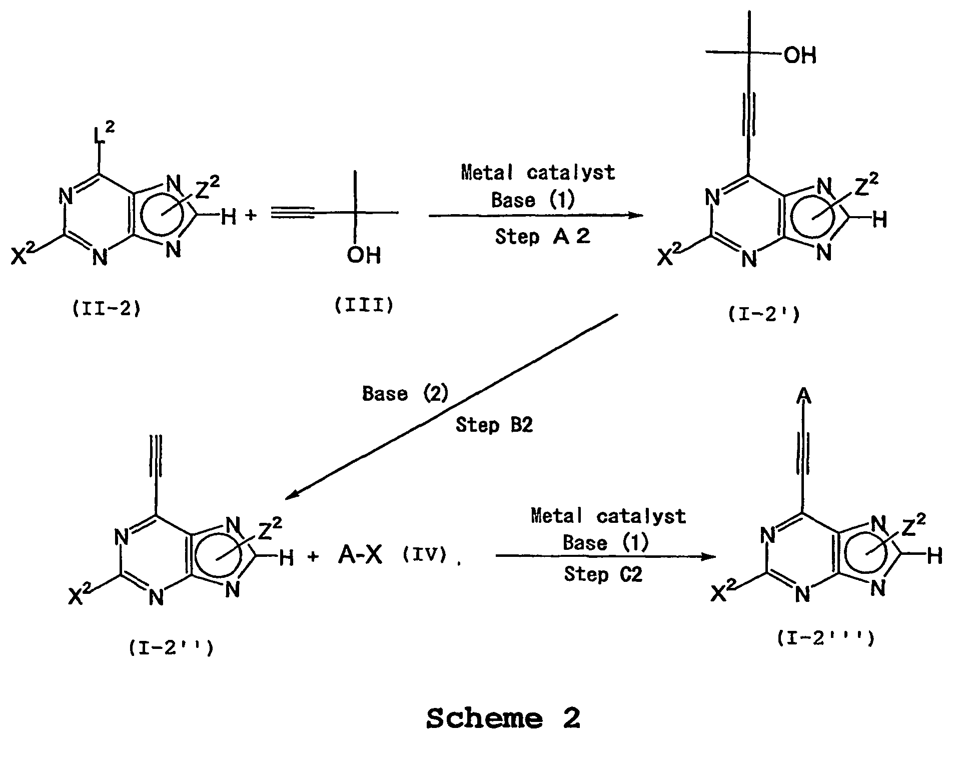 In the Scheme, each symbol is as defined above. 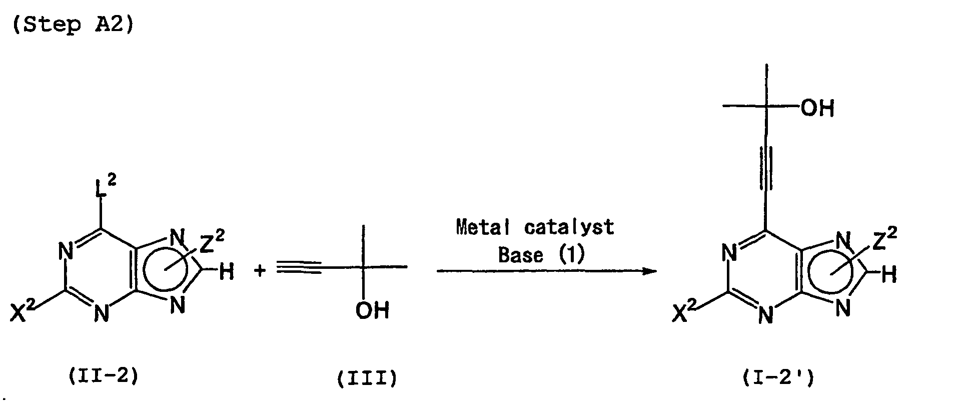 wherein each symbol is as defined above.

In Step A2, a compound represented by the formula (I-2') (hereinafter sometimes to be referred to as compound (I-2')) or a salt thereof is obtained by reacting a compound represented by the formula (II-2) wherein L² is a halogen atom (hereinafter sometimes to be referred to as compound (II-2)) or a salt thereof with compound (III), in the presence of a metal catalyst and a base (1).

In Step A2, a palladium compound may be used alone as a metal catalyst, as defined above. When a palladium compound is to be used alone, the palladium compound is particularly preferably bis(triphenylphosphine)palladium dichloride. The amount of the palladium compound to be used is not particularly limited as long as a catalytic amount is used relative to compound (II-2) or a salt thereof, and is generally 0.01-1.0 mmole, preferably 0.05-0.2 mmole, per 1 mmole of compound (II-2) or a salt thereof. As a metal catalyst, a combination of a palladium compound and a copper compound is preferable. When a palladium compound and a copper compound are used in combination, bis(triphenylphosphine)palladium dichloride is particularly preferable as a palladium compound, and cuprous iodide is particularly preferable as a copper compound. The amount of the palladium compound to be used and the amount of the copper compound to be used are not particularly limited as long as a catalytic amount is used relative to compound (II-2) or a salt thereof, and the amount of the palladium compound to be used is generally 0.01-1.0 mmole, preferably 0.05-0.2 mmole, per 1 mmole of compound (II-2) or a salt thereof, and the amount of the copper compound to be used is generally 0.01-1.0 mmole, preferably 0.05-0.2 mmole, per 1 mmole of compound (II-2) or a salt thereof.

In Step A2, an amine compound is preferably used as a base (1), as defined above. As the amine compound, trialkylamine is preferable. As the trialkylamine, triethylamine and ethyldiisopropylamine are preferable. The amount of the base (1) to be used is generally 1.0-5.0 mmole, preferably 1.0-1.5 mmole, per 1 mmole of compound (II-2) or a salt thereof.

The salt of compound (II-2) used in Step A2 is not particularly limited and, for example, a salt of compound (II-2) with an organic acid (e.g., methanesulfonic acid, trifluoromethanesulfonic acid, p-toluenesulfonic acid etc.), a salt of compound (II-2) with an inorganic acid (e.g., hydrochloric acid, sulfuric acid, phosphoric acid, nitric acid etc.), ammonium salt thereof (e.g., tetraalkylammonium salt etc.) and the like are mentioned.

In Step A2, the amount of compound (III) to be used is, generally 1.0-5.0 mmole, preferably 1.0-1.5 mmole, per 1 mmole of compound (II-2) or a salt thereof.

In Step A2, the solvent is not particularly limited as long as the reaction is not adversely affected by the solvent, and, for example, aprotic solvents such as DMF, DMSO, N,N-dimethylacetamide, THF and the like, and the like are mentioned, with preference given to DMF. The amount of the solvent to be used is not particularly limited, and is generally 0.5-10 mL, preferably 1-5 mL, per 1 mmole of compound (II-2) or a salt thereof.

In Step A2, the reaction time varies depending on the reagents and the solvent to be used, and is generally 1-5 hrs.

In Step A2, the reaction temperature varies depending on the reagents and the solvent to be used, and is generally 40°C-150°C, preferably 80°C-100°C.

As the salt of compound (I-2'), the salts recited above are mentioned. 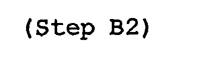 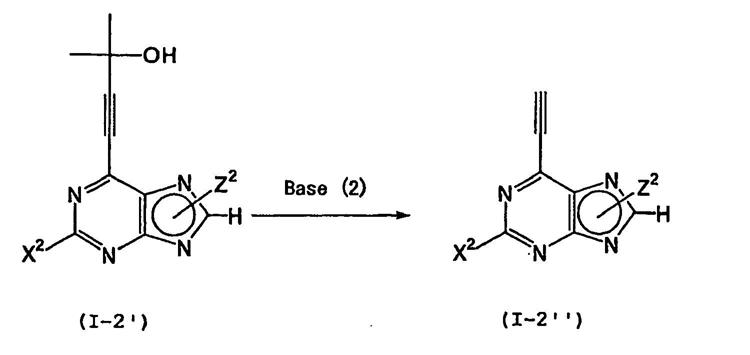 wherein each symbol is as defined above.

In Step B2, a compound represented by the formula (I-2") (hereinafter sometimes to be referred to as compound (I-2")) or a salt thereof is obtained by treating compound (I-2') obtained in the above-mentioned Step A2 or a salt thereof with a base (2).

In Step B2, alkali metal hydroxide is particularly preferably used as the base (2), as defined above, with preference given to sodium hydroxide and potassium hydroxide.

In Step B2, the amount of base (2) to be used is generally 1-20 mmole, preferably 3-10 mmole, per 1 mmole of compound (I-2') or a salt thereof.

In Step B2, the solvent is not particularly limited as long as the reaction is not adversely affected by the solvent, and, for example, aprotic solvents such as toluene, xylene, THF, MTBE and the like, and the like are mentioned, with preference given to toluene. The amount of the solvent to be used is not particularly limited, and is generally 10-500 mL, preferably 50-200 mL, per 1 mmole of compound (I-2') or a salt thereof.

In Step B2, the reaction time varies depending on the reagents and the solvent to be used, and is generally 1-5 hrs.

In Step B2, the reaction temperature varies depending on the reagents and the solvent to be used, and is generally 60°C-150°C, preferably 90°C-110°C.

As the salt of compound (I-2"), those similar to the salts recited above are mentioned. 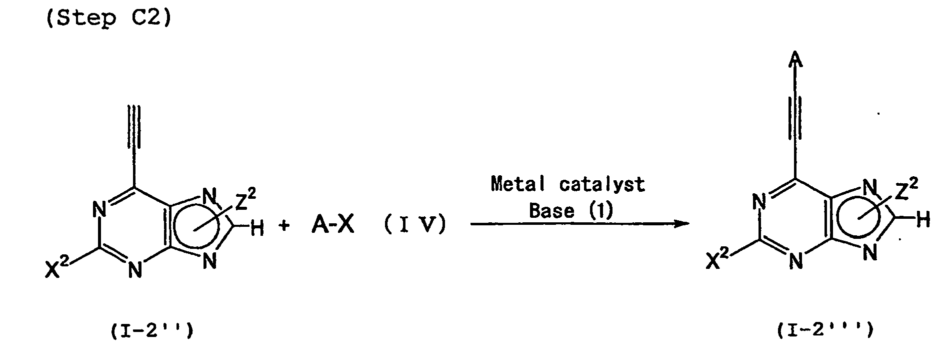 wherein each symbol is as defined above.

In Step C2, a compound represented by the formula (I-2"') (hereinafter sometimes to be referred to as compound (I-2"')) or a salt thereof is obtained by reacting compound (I-2") obtained in the above-mentioned Step B2 or a salt thereof with compound (IV), in the presence of a metal catalyst and a base (1).

In Step C2, a palladium compound may be used alone as a metal catalyst, as defined above. When a palladium compound is used alone, bis(triphenylphosphine)palladium dichloride is particularly preferable as the palladium compound. The amount of the palladium compound to be used is not particularly limited as long as a catalytic amount is used, relative to compound (I-2") or a salt thereof. It is generally 0.01-1.0 mmole, preferably 0.05-0.2 mmole, per 1 mmole of compound (I-2") or a salt thereof. As a metal catalyst, a combination of a palladium compound and a copper compound is preferable. When a palladium compound and a copper compound are used in combination, the palladium compound is particularly preferably bis(triphenylphosphine)palladium dichloride and the copper compound is particularly preferably cuprous iodide. The amount of the palladium compound to be used and the amount of the copper compound to be used are not particularly limited as long as a catalytic amount is used relative to compound (I-2") or a salt thereof. The amount of the palladium compound to be used is generally 0.01-1.0 mmole, preferably 0.05-0.2 mmole, per 1 mmole of compound (I-2") or a salt thereof, and the amount of the copper compound to be used is generally 0.01-1.0 mmole, preferably 0.05-0.2 mmole, per 1 mmole of compound (I-2") or a salt thereof.

In Step C2, an amine compound is preferably used as a base (1), as defined above, and as the amine compound, trialkylamine is preferable. As the trialkylamine, triethylamine and ethyldiisopropylamine are preferable. The amount of the base (1) to be used is generally 1.0-5.0 mmole, preferably 1.0-3.0 mmole, per 1 mmole of compound (I-2") or a salt thereof.

In Step C2, the amount of the compound (IV) to be used is generally 1.0-2.0 mmole, preferably 1.0-1.3 mmole, per 1 mmole of compound (1-2") or a salt thereof.

In Step C2, the solvent is not particularly limited as long as the reaction is not adversely affected by the solvent, and, for example, aprotic solvents such as DMF, N,N-dimethylacetamide, DMSO, THF and the like, and the like are mentioned, with preference given to DMF. The amount of the solvent to be used is not particularly limited, and is generally 1-200 mL, preferably 5-20 mL, per 1 mmole of compound (I-2") or a salt thereof.

In Step C2, the reaction time varies depending on the reagents and solvent to be used, and is generally 2-8 hrs.

In Step C2, the reaction temperature varies depending on the reagents and solvent to be used, and is generally 50°C-150°C, preferably 80°C-100°C.

As the salt of compound (I-2"'), those similar to the salts recited above are mentioned.

Accordingly, the alkynylpurine compound represented by the formula (I-2) is considered to encompass compounds (I-2'), (I-2"), (I-2"') (including isomers such as tautomer and the like, obtainable in each step) and salts thereof obtained by the reactions described in Scheme 2, as well as compounds and salts thereof obtained by the methods according to Scheme 2.

The above-mentioned Steps A1-C1 and A2-C2 may further include steps of protecting a hydroxy group and an amino group with a suitable protecting group as necessary, and removing the protecting group, by a known means for those of ordinary skill in the art of the organic synthesis.

In the above-mentioned Steps A1-C1 and A2-C2, each product may be isolated and/or purified by an isolation or purification means known to those of ordinary skill in the art of the organic synthesis (e.g., crystallization, recrystallization, distillation, chromatography etc.).

As described above, according to the present invention, an alkynylpurine compound represented by the formula (I) (specifically, compounds of the formula (I-1) and the formula (I-2)) and salts thereof can be produced.

Of the alkynylpurine compounds represented by the formula (I), a compound wherein the alkynyl moiety is Me₂(OH)C-C≡C- is preferable as an intermediate for the production of medicaments. The reason therefor is that the alkynyl moiety [Me₂(OH)C-C≡C-] can be easily converted to ethynyl [HC≡C-] by a treatment with a base (2) in the above-mentioned Step B1 or B2 (i.e., elimination of acetone via β-elimination). Of the alkynylpurine compounds wherein the alkynyl moiety is Me₂(OH)C-C≡C-, the following three compounds (having amino-protecting group such as tetrahydropyran-2-yl, benzyl and the like where necessary) are particularly preferable.

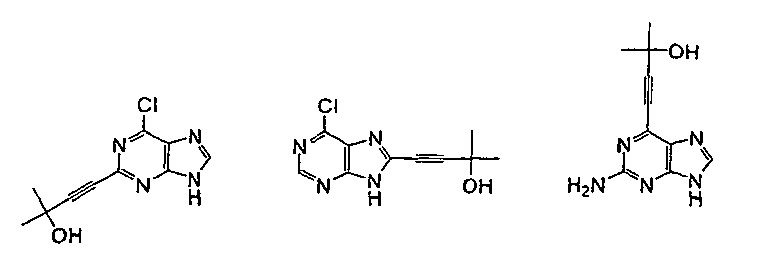

In addition, the compound according to the present invention wherein the alkynyl moiety is ethynyl [HC≡C-] can lead many alkynylpurine compounds wherein the alkynyl moiety is A-C≡C- (A is as defined above) by further modification of the ethynyl (see the above-mentioned Step C). A greater number of alkynylpurine derivatives can be also provided by further modification of A, X¹ or X², Z¹ or Z² of compound (I-1"') or compound (I-2"').

Accordingly, the present invention is characterized in that various alkynylpurine compounds can be derived via an ethynylpurine compound (i.e., compound (I-1) and (I-2) having an alkynyl moiety (R-C≡C-) wherein R is hydrogen atom) as a key intermediate. As the ethynylpurine compound, particularly the following three compounds (having an amino-protecting group, as necessary, such as tetrahydropyran-2-yl, benzyl and the like) are preferable.

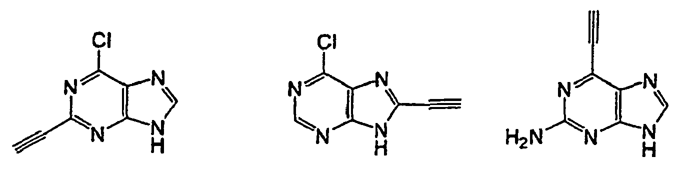

The present invention is explained in detail by referring to the following Examples. These Examples are given for exemplification purposes alone and do not limit the present invention in any way.

### Reference Example 1

Synthesis of 4-(6-chloropurin-2-yl)-2-methylbutan-3-yn-2-ol 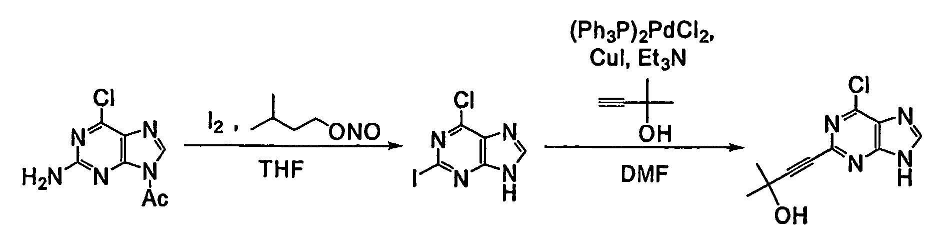
(1) 9-Acetyl-2-amino-6-chloro-9H-purine (10.0 g, 0.047 mole), iodine (9.0 g, 0.036 mole) and isoamyl nitrite (20.0 g, 0.171 mole) were mixed in THF (100 mL) and heated at 50-60°C to allow reaction. After cooling to room temperature, a 15% aqueous sodium thiosulfate solution (100 mL) was added to the obtained reaction mixture and the mixture was extracted three times with methylisobutylketone (50 mL). The obtained organic layers were combined and extracted three times with 5% aqueous sodium hydroxide solution (100 mL). This alkaline aqueous solution was adjusted to pH 4-5 to give yellow crystals. The crystals were collected by filtration and dried under reduced pressure to give 6-chloro-2-iodo-9H-purine (10.0 g, 0.036 mole, yield 76%).
   Analytical data
   ¹H-NMR (400 MHz, DMSO-d₆) δ (ppm) : 8.57 (s, 1H).
   ¹³C-NMR (100 MHz, DMSO-d₆) δ (ppm): 116.6, 129.5, 146.3, 147.8, 156.6.
   MS (EI) m/z: 282 (M⁺, 32), 280 (M⁺, 100), 155 (32), 153 (94).
(2) 6-chloro-2-iodo-9H-purine (2.80 g, 10 mmole), triethylamine (1.21 g, 12 mmole), bis(triphenylphosphine)palladium dichloride (0.30 g, 0.5 mmole), 2-methylbutan-3-yn-2-ol (1.01 g, 12 mmole) and cuprous iodide (0.10 g, 0.5 mmole) were mixed in DMF (20 mL) and the mixture was heated at 90°C for 5 hrs. under a nitrogen atmosphere. DMF was evaporated under reduced pressure and the obtained residue was applied to silica gel column chromatography to give the objective 4-(6-chloro-9H-purin-2-yl)-2-methylbutan-3-yn-2-ol (1.36 g, 5.74 mmole, yield 57%).
   Analytical data
   ¹H-NMR (400 MHz, DMSO-d₆) δ (ppm) : 1.48 (s, 6H), 5.68 (br, 1H), 8.70 (s, 1H), 13.93 (br, 1H).
   ¹³C-NMR (100 MHz, DMSO-d₆) δ (ppm): 30.9, 63.4, 79.5, 92.8, 129.5, 144.0, 146.6, 148.1, 153.1.
   MS (EI) m/z: 238 (M⁺, 6.5), 236 (M⁺, 17), 223 (28), 221 (100), 195 (20), 193 (61), 181 (12), 179 (34), 143 (27).

### Reference Example 2

### Synthesis of 4-[6-chloro-9-(tetrahydropyran-2-yl)-9H-purin-2-yl]-2-methylbutan-3-yn-2-ol

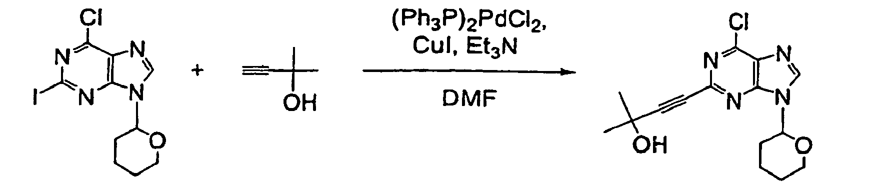

6-Chloro-2-iodo-9-(tetrahydropyran-2-yl)-9H-purine (2.80 g, 7.8 mmole), triethylamine (0.96 g, 9.5 mmole), bis(triphenylphosphine)palladium dichloride (0.28 g, 0.40 mmole), 2-methylbutan-3-yn-2-ol (0.83 g, 9.9 mmole) and cuprous iodide (0.10 g, 0.53 mmole) were mixed in DMF (15 mL) and the mixture was heated at 90°C for 5 hrs. under a nitrogen atmosphere. DMF was evaporated under reduced pressure and the obtained reaction mixture was subjected to silica gel column chromatography to give the objective 4-[6-chloro-9-(tetrahydropyran-2-yl)-9H-purin-2-yl]-2-methylbutan-3-yn-2-ol (1.50 g, 4.7 mmole, yield 60%).
Analytical data
¹H-NMR (400 MHz, DMSO-d₆) δ (ppm): 1.53-1.70 (m, 8H), 1.75-1.85 (m, 1H), 1.97-2.11 (m, 2H), 2.25-2.29 (m, 1H), 3.73-3.81 (m, 1H), 4.02-4.07 (m, 1H), 5.72-5.83 (m, 2H), 8.96 (s, 1H). ¹³C-NMR (100 MHz, DMSO-d₆) δ (ppm): 22.0, 24.4, 29.8, 31.0, 63.4, 67.7, 79.3, 81.4, 93.7, 130.0, 144.2, 146.0, 148.8, 151.1.
MS (EI) m/z: 322 (M⁺, 1. 2), 320 (M⁺, 4.0), 239 (2. 0), 237 (11.9), 85 (100).

### Example 1

### Synthesis of 6-chloro-2-ethynyl-9-(tetrahydropyran-2-yl)-9H-purine

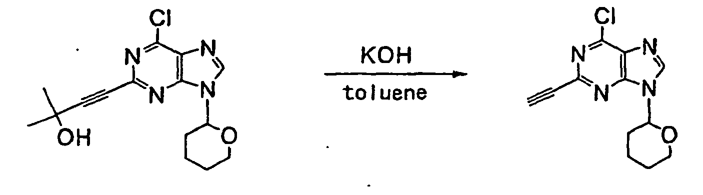

4-[6-Chloro-9-(tetrahydropyran-2-yl)-9H-purin-2-yl]-2-methylbutan-3-yn-2-ol (0.15 g, 0.47 mmole) and potassium hydroxide (0.15 g, 2.6 mmole) were added to toluene (30 mL) and the mixture was heated under reflux for 3 hrs. The reaction mixture was cooled to room temperature and washed with water (30 mLx2). Toluene was evaporated under reduced pressure to give the objective 6-chloro-2-ethynyl-9-(tetrahydropyran-2-yl)-9H-purine (0.10 g, 0.38 mmole, yield 81%).
Analytical data
¹H-NMR (400 MHz, DMSO-d₆) δ (ppm): 1.60-1.80 (m, 3H), 1.90-2.02 (m, 2H), 2.24-2.29 (m, 1H), 3.72-3.78 (m, 1H), 4.02-4.04 (m, 1H), 4.51 (s, 1H), 5.75-5.79 (m, 1H), 8.86 (s, 1H).
¹³C-NMR (100 MHz, DMSO-d₆) δ (ppm): 22.1, 24.4, 29.8, 67.7, 78.9, 81.5, 90.1, 130.7, 143.4, 146.5, 148.9, 151.1.
MS (EI) m/z: 264 (M⁺, 2.8), 262 (M⁺, 8.7), 234 (3.7), 181 (4.1), 179 (19), 85 (100).

### Reference Example 3

### Synthesis of 4-[6-chloro-9-(tetrahydropyran-2-yl)-9H-purin-8-yl]-2-methylbutan-3-yn-2-ol

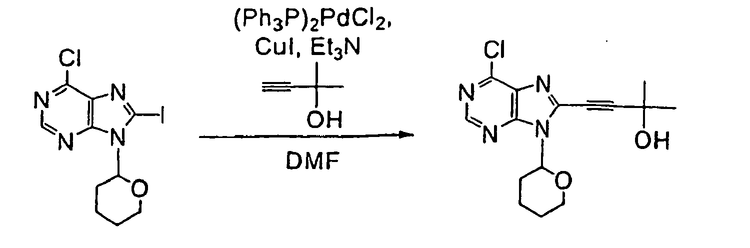

6-Chloro-8-iodo-9-(tetrahydropyran-2-yl)-9H-purine (0.8 g, 2.2 mmole) synthesized according to the method described in Synth. Commun., 1998, 28 (23), 4303-4315, 2-methylbutan-3-yn-2-ol (0.28 g, 3.3 mmole), triethylamine (0.30 g, 3.0 mmole), bis(triphenylphosphine)palladium dichloride (0.14 g, 0.20 mmole) and cuprous iodide (0.06 g, 0.32 mmole) were mixed in DMF (100 mL), and the mixture was heated at 80°C for 3 hrs. under a nitrogen atmosphere. DMF was evaporated under reduced pressure and the obtained reaction mixture was added to an aqueous ammonium chloride solution. The mixture was extracted with methylisobutylketone and the organic layer was dried over anhydrous magnesium sulfate. The organic solvent was evaporated, and the obtained residue was separated and purified by silica gel column chromatography to give the objective 4-[6-chloro-9-(tetrahydropyran-2-yl)-9H-purin-8-yl]-2-methylbutan-3-yn-2-ol (0.30 g, 0.94 mmole, yield 43%).
Analytical data
¹H-NMR (400 MHz, DMSO-d₆) δ (ppm) : 1.60 (s, 6H), 1.60-1. 80 (m, 3H), 1.90-2.10 (m, 2H), 2.85-2.95 (m, 1H), 3.70-3.76 (m, 1H), 4.10-4.15 (m, 1H), 5.80-5.85 (m, 1H), 5.99 (br, 1H), 8.84 (s, 1H).
¹³C-NMR (100 MHz, DMSO-d₆) δ (ppm): 22.6, 24.4, 28.4, 30.6, 63.8, 68.2, 70.2, 83.4, 104.3, 130.3, 138.4, 149.0, 150.9, 152.2.
MS (EI) m/z: 322 (M⁺, 1.6), 320 (M⁺, 5.2), 239 (5.7), 237 (37), 85 (100).

### Example 2

### Synthesis of 6-chloro-8-ethynyl-9-(tetrahydropyran-2-yl)-9H-purine

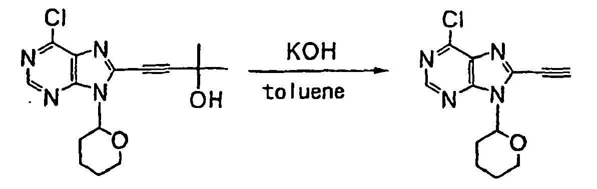

4-[6-Chloro-9-(tetrahydropyran-2-yl)-9H-purin-8-yl]-2-methylbutan-3-yn-2-ol (0.10 g, 0.31 mmole) and potassium hydroxide (0.10 g, 1.78 mmole) were added to toluene (20 mL) and the mixture was heated at 80°C for 3 hrs. The reaction mixture was cooled to room temperature and washed with water (20 mLx2). Toluene was evaporated under reduced pressure and the obtained residue was subjected to column chromatography to give the objective 6-chloro-8-ethynyl-9-(tetrahydropyran-2-yl)-9H-purine (0.06 g, 0.27 mmole, yield 74%).
Analytical data
¹H-NMR (400 MHz, DMSO-d₆) δ (ppm): 1.60-1.80 (m, 3H), 1.90-2.10 (m, 2H), 2.85-2.90 (m, 1H), 3.67-3.75 (m, 1H), 4.02-4.10 (m, 1H), 5.28 (s, 1H), 5.80-5.85 (m, 1H), 8.86 (s, 1H).
¹³C-NMR (100 MHz, DMSO-d₆) δ (ppm): 22.4, 24.4, 28.4, 68.2, 72.4, 83.6, 90.1, 130.3, 137.7, 149.5, 150.9, 152.6.
MS (EI) m/z: 264 (M⁺, 2.3), 262 (M⁺, 5.4), 181 (6.8), 179 (19), 85 (100).

### Example 3

### Synthesis of 4-(2-amino-9-benzyl-9H-purin-6-yl)-2-methylbutan-3-yn-2-ol

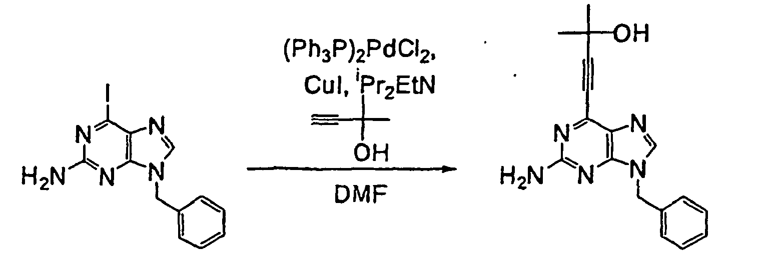

2-Amino-9-benzyl-6-iodo-9H-purine (1.8 g, 5.1 mmole), 2-methylbutan-3-yn-2-ol (0.50 g, 5.9 mmole), ethyldiisopropylamine (0.78 g, 6.0 mmole), bis(triphenylphosphine)palladium dichloride (0.18 g, 0.26 mmole) and cuprous iodide (0.048 g, 0.25 mmole) were mixed in DMF (20 mL), and the mixture was heated at 80°C for 3 hrs. under a nitrogen atmosphere. After cooling to room temperature, a saturated aqueous ammonium chloride solution (50 mL) was added to the mixture and the mixture was extracted with methylisobutylketone (50 mL×3). The organic layer was dried over anhydrous magnesium sulfate. The organic solvent was evaporated, and the obtained residue was separated and purified by silica gel column chromatography to give the objective 4-(2-amino-9-benzyl-9H-purin-6-yl)-2-methylbutan-3-yn-2-ol (1.0 g, 3.3 mmole, yield 66%).
Analytical data
¹H-NMR (400 MHz, DMSO-d₆) δ (ppm): 1.48 (s, 6H), 5.28 (s, 2H), 5.68 (s, 1H), 6.60 (br s, 2H), 7.22-7.34 (m, 5H), 8.19 (s, 1H).
¹³C-NMR (100 MHz, DMSO-d₆) δ (ppm): 31.18, 45.67, 63.63, 76.52, 101.40, 126.95, 127.52, 128.54, 136.68, 140.97, 142.97, 153.42, 160.23.
MS (EI) m/z: 307 (M⁺).

### Example 4.

### Synthesis of 2-amino-9-benzyl-6-ethynyl-9H-purine

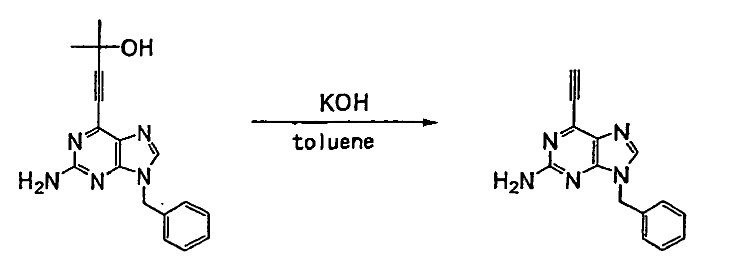

4-(2-Amino-9-benzyl-9H-purin-6-yl)-2-methylbutan-3-yn-2-ol (50.0 mg, 0.148 mmole) and potassium hydroxide (50 mg, 0.89 mmole) were added to toluene (10 mL) and the mixture was heated under reflux for 5 hrs. The reaction mixture was cooled to room temperature and washed with water (10 mL×2). Toluene was evaporated under reduced pressure to give the objective product, 2-amino-9-benzyl-6-ethynyl-9H-purine (34.0 mg, 0.136 mmole, yield 92%).
Analytical data
¹H-NMR (400 MHz, DMSO-d₆) δ (ppm) : 4.22 (s, 1H), 5.30 (s, 2H), 6.33 (br, 2H), 7.24-7.34 (m, 5H), 7.37 (dd, 1H), 8.05 (s, 1H). ¹³C-NMR (100 MHz, DMSO-d₆) δ (ppm): 45.7, 78.4, 84.9, 126.8, 127.3, 127.8, 128.2, 135.9, 140.1, 142.6, 153.3, 159.9.
MS (EI, 70 eV) m/z: 249 (M⁺, 24), 91 (100).

### Example 5

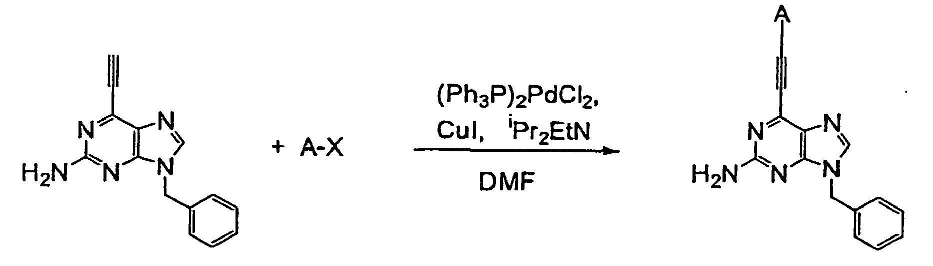

Halide: A-X (0.39 mmole), bis(triphenylphosphine)palladium dichloride (13 mg, 0.018 mmole), ethyldiisopropylamine (0.19 g, 1.08 mmole) and cuprous iodide (3.4 mg, 0.018 mmole) were added to DMF (3 mL). Thereto was added a solution (2 mL) of 2-amino-9-benzyl-6-ethynyl-9H-purine (100 mg, 0.36 mmole) in DMF under a nitrogen atmosphere. The obtained reaction mixture was heated at 80°C (see the following Table 1 for reaction time (Time (h))). The organic solvent was evaporated under reduced pressure and the residue was dissolved in THF (5 mL). The solution was washed with aqueous ammonia and THF was evaporated under reduced pressure. The residue was purified by silica gel chromatography to give the corresponding 2-amino-9-benzyl-6-(substituted ethynyl)-9H-purine (see the following Table 1 for Yield (%)).

**Table 1**

| | | | | | | | |
|---|---|---|---|---|---|---|---|
| 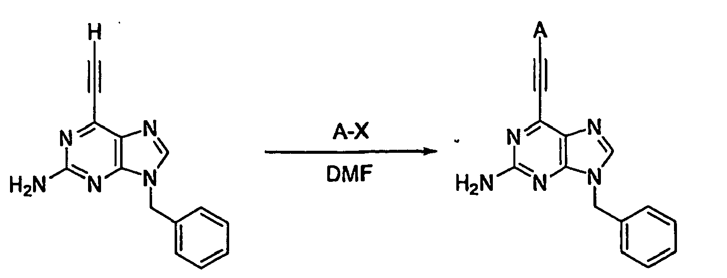 | | | | | | | |

| **A** | **X** | **Time (h)** | **Yield (%)** | **A** | **X** | **Time (h)** | **Yield (%)** |
|---|---|---|---|---|---|---|---|
| 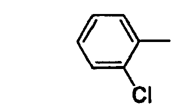 | | 8 | 58 | 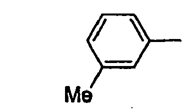 | I | 8 | 45 |
| 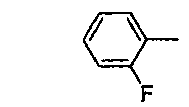 | | 8 | 63 | 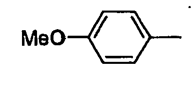 | I | 8 | 71 |
| 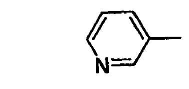 | | 8 | 65 | 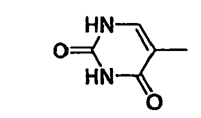 | I | 8 | 34 |
| 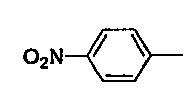 | | 8 | 91 | 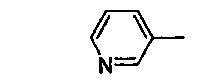 | Br | 8 | 30 |

2-Amino-9-benzyl-6-(2-chlorophenylethynyl)-9H-purine
Analytical data
¹H-NMR (500 MHz, CDCl₃) δ (ppm): 5.21 (s, 2H), 5.29 (br, 2H), 7.17-7.32 (m, 7H), 7.37 (dd, 1H), 7.65 (dd, 1H), 7.76 (s, 1H). ¹³C-NMR (125 MHz, CDCl₃) δ (ppm): 45.8, 87.2, 92.8, 120.6, 125.5, 126.7, 127.5, 127.9, 128.1, 128.4, 129.7, 133.3, 134.2, 135.8, 140.3, 142.2, 153.0, 158.6.
MS (EI, 70 eV) m/z: 361 (M⁺, 2), 359 (M⁺, 7), 277 (81), 183 (45), 91 (100).
2-Amino-9-benzyl-6-(2-fluorophenylethynyl)-9H-purine
Analytical data
¹H-NMR (500 MHz, DMSO-d₆) δ (ppm): 5.25 (s, 2H), 6.67 (br, 2H), 7.16-7.35 (m, 7H), 7.50 (dd, 1H), 7.64 (s, 1H), 8.28 (s, 1H). ¹³C-NMR (125 MHz, DMSO-d₆) δ (ppm): 45.3, 86.8, 89.2, 108.8, 108.9, 115.4, 115.5, 124.5, 126.6, 127.2, 128.2, 132.0, 133.4, 136.2, 139.7, 160.0, 160.7, 162.7.
MS (EI, 70 eV) m/z: 343 (M⁺, 23), 91 (100), 57 (36).
2-Amino-9-benzyl-6-(pyridin-3-ylethynyl)-9H-purine
Analytical data
¹H-NMR (500 MHz, CDCl₃) δ (ppm) : 5.23 (s, 2H), 5.41 (br, 2H), 7.19-7.23 (m, 2H), 7.28-7.35 (m, 4H), 7.78 (s, 1H), 7.97 (d, 1H), 8.58 (d, 1H), 8.88 (s, 1H) .
¹³C-NMR (125 MHz, CDCl₃) δ (ppm) : 46.0, 84.2, 85.0, 122.1, 126.7, 127.7, 128.2, 128.4, 138.7, 142.8, 149.0, 152.0, 157.9.
2-Amino-9-benzyl-6-(4-nitrophenylethynyl)-9H-purine
Analytical data
¹H-NMR (500 MHz, DMSO-d₆) δ (ppm) : 5.32 (s, 2H), 7.22-7.32 (m, 5H), 7.85 (s, 1H), 8.09 (d, 2H), 8.28 (s, 2H), 8.77 (s, 1H) . ¹³C-NMR (125 MHz, DMSO-d₆) δ (ppm): 47.4, 121.6, 123.6, 124.9, 128.4, 128.9, 129.3, 129.7, 136.6, 138.8, 143.1, 149.2, 158.7. MS (EI, 70 eV) m/z: 339 (7), 277 (14), 149 (100), 57 (48).
2-Amino-9-benzyl-6-(3-methylphenylethynyl)-9H-purine
Analytical data
¹H-NMR (250 MHz, CDCl₃) δ (ppm): 2.35 (s, 3H), 5.24 (s, 2H), 5.98 (br, 2H), 7.24-7.35 (m, 7H), 7.53 (d, 1H), 7.58 (s, 1H), 7.86 (s, 1H).
¹³C-NMR (62.5 MHz, CDCl₃) δ (ppm): 21.0, 46.9, 83.3, 99.5, 121.0, 127.7, 128.3, 128.5, 129.1, 129.9, 130.9, 133.3, 134.9, 138.1, 140.8, 143.1, 145.5, 154.1, 159.8.
MS (EI, 70 eV) m/z: 339 (M⁺, 14), 277 (24), 149 (100), 91 (49), 57 (48).
2-Amino-9-benzyl-6-(4-methoxyphenylethynyl)-9H-purine
Analytical data
¹H-NMR (500 MHz, CDCl₃) δ (ppm): 3.75 (s, 3H), 5.18 (s, 2H), 5.41 (br, 2H), 6.81 (d, 2H), 7.15-7.29 (m, 5H), 7.59 (d, 2H), 7.72 (s, 1H).
¹³C-NMR (125 MHz, CDCl₃) δ (ppm): 45.8, 54.3, 82.1, 97.4, 113.1, 126.6, 127.4, 128.1, 131.0, 133.5, 134.3, 141.3, 141.6, 152.6, 158.8, 159.9.
MS (EI, 70 eV) m/z: 355 (M⁺, 10), 281 (34), 277 (19), 147 (43), 57 (100).
5-[(2-Amino-9-benzyl-9H-purin-6-yl)ethynyl]-1H-pyrimidine-2,4-dion
Analytical data
¹H-NMR (250 MHz, DMSO-d₆) δ (ppm): 5.29 (s, 2H), 6.72 (br, 2H), 7.25-7.31 (m, 5H), 8.03 (s, 1H), 8.21 (s, 1H), 11.74 (br, 2H). ¹³C-NMR (62.5 MHz, DMSO-d₆) δ (ppm): 47.4, 85.4, 100.4, 113.4, 117.8, 119.0, 127.1, 128.7, 134.7, 137.2, 147.0, 155.6, 159.1, 163.9, 172.5, 174.0.

### Industrial Applicability

According to the present invention, a method for producing an alkynylpurine compound and a salt thereof useful as an intermediate for medicament production safely, conveniently and economically from the corresponding purine compound and a salt thereof, as well as an alkynylpurine compound and a salt thereof useful as an intermediate for medicament production can be provided.

This application is based on a patent application No. 2002-175015 filed in Japan, the contents of which are hereby incorporated by reference.

## Claims

1. A compound represented by the formula (I-1): 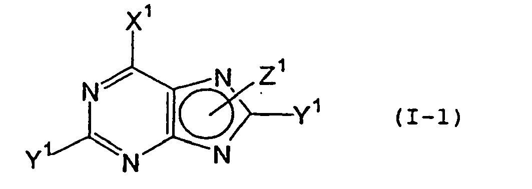 wherein
X¹ is an alkyl group, an alkoxy group, an aryl group, an optionally protected amino group, a halogen atom or a hydrogen atom,
one of Y¹ is a group represented by the formula: R-C≡C-wherein R is a hydrogen atom, and the other Y¹ is a hydrogen atom, and
Z¹ is an alkyl group, an amino-protecting group or a hydrogen atom, which is attached to a nitrogen atom at the 7- or 9-position of the purine nucleus,
or a salt thereof.

2. The compound of claim 1, wherein X¹ is a halogen atom, and Z¹ is an amino-protecting group or a hydrogen atom.

3. The compound of claim 1 or 2, wherein X¹ is a chlorine atom.

4. The compound of any of claims 1 to 3, wherein Z¹ is tetrahydropyran-2-yl, benzyl or a hydrogen atom.

5. A compound represented by the formula (I-2): 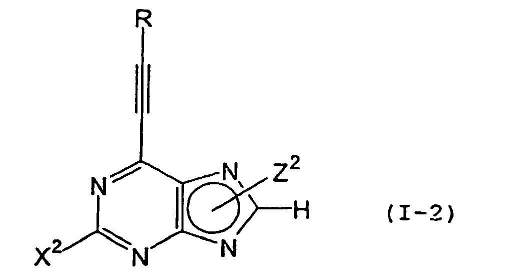 wherein
R is a hydrogen atom or a hydrocarbon group optionally having substituents,
X² is an alkyl group, an alkoxy group, an aryl group, an optionally protected amino group or a halogen atom, and
Z² is an alkyl group, an amino-protecting group or a hydrogen atom, which is attached to a nitrogen atom at the 7- or 9-position of the purine nucleus,
or a salt thereof.

6. The compound of claim 5, wherein R is a hydrogen atom or Me₂(OH)C-, X² is an optionally protected amino group, and Z² is an amino-protecting group or a hydrogen atom.

7. The compound of claim 5 or 6, wherein Z² is tetrahydropyran-2-yl, benzyl or a hydrogen atom.

8. A production method of a compound represented by the formula (I-1"): 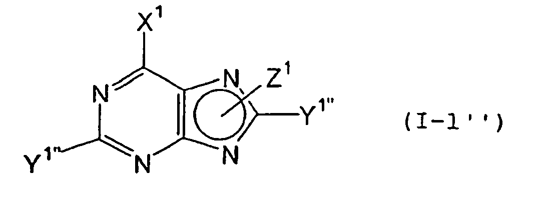 wherein
X¹ is an alkyl group, an alkoxy group, an aryl group, an optionally protected amino group, a halogen atom or a hydrogen atom,
Z¹ is an alkyl group, an amino-protecting group or a hydrogen atom, which is attached to a nitrogen atom at the 7- or 9-position of the purine nucleus, and
one of Y^{1"} is a group represented by the formula: HC≡C-, and the other Y^{1"} is a hydrogen atom, or a salt thereof, which comprises treating a compound represented by the formula (I-1') : 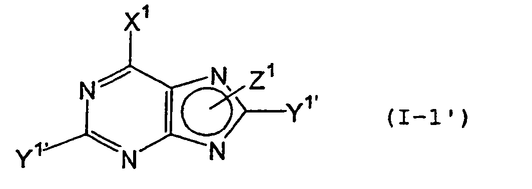
wherein
X¹ and Z¹ are as defined above, and
one of Y^{1'} is a group represented by the formula: Me₂(OH) C-C≡C-, and the other Y^{1'} is a hydrogen atom,
or a salt thereof, with a base (2).

9. A production method of a compound represented by the formula (I-1"'): 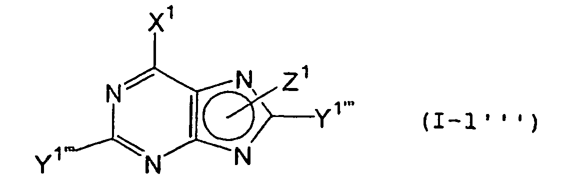 wherein
X¹ is an alkyl group, an alkoxy group, an aryl group, an optionally protected amino group, a halogen atom or a hydrogen atom,
Z¹ is an alkyl group, an amino-protecting group or a hydrogen atom, which is attached to a nitrogen atom at the 7- or 9-position of the purine nucleus, and
one of Y^{1"'} is a group represented by the formula: A-C≡C-, wherein A is an aryl group optionally having substituents or a heterocyclic group optionally having substituents, and the other Y^{1"'} is a hydrogen atom,
or a salt thereof, which comprises reacting a compound represented by the formula (I-1") : 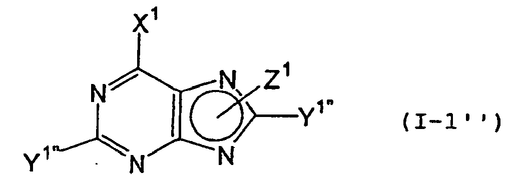
wherein
X¹ and Z¹ are as defined above, and
one of Y^{1"} is a group represented by the formula: HC≡C-, and the other Y^{1"} is a hydrogen atom,
or a salt thereof, with a compound represented by the formula (IV): A-X wherein A is as defined above, and X is a halogen atom, in the presence of a metal catalyst and a base (1).

10. A production method of a compound represented by the formula (I-2"'): 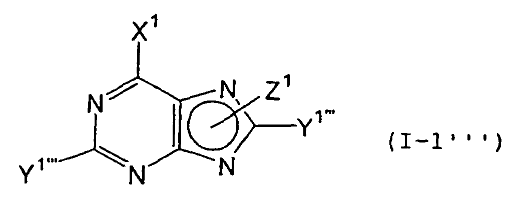 wherein
X¹ is an alkyl group, an alkoxy group, an aryl group, an optionally protected amino group, a halogen atom or a hydrogen atom,
one of Y^{1"'} is a group represented by the formula: A-C≡C-,
wherein A is an aryl group optionally having substituents or a heterocyclic group optionally having substituents, and the other Y^{1"'} is a hydrogen atom, and
Z¹ is an alkyl group, an amino-protecting group or a hydrogen atom, which is attached to a nitrogen atom at the 7- or 9-position of the purine nucleus,
or a salt thereof, which comprises the following steps (a)-(c) :
(a) a step of obtaining a compound represented by the formula (I-1') : 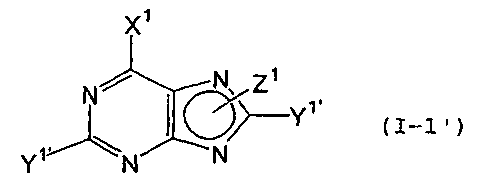 wherein
X¹ is an alkyl group, an alkoxy group, an aryl group, an optionally protected amino group, a halogen atom or a hydrogen atom,
Z¹ is an alkyl group, an amino-protecting group or a hydrogen atom, which is attached to a nitrogen atom at the 7- or 9-position of the purine nucleus, and
one of Y^{1'} is a group represented by the formula: Me₂(OH)C-C≡C-, and the other Y^{1'} is a hydrogen atom,
or a salt thereof, which comprises reacting a compound represented by the formula (II-1) : 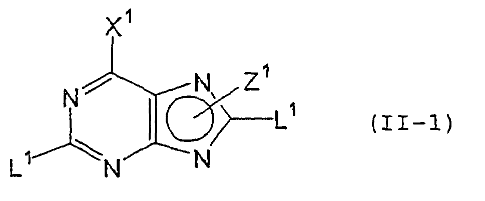
wherein
X¹ and Z¹ are as defined above, and
one of L¹ is a halogen atom, and the other L¹ is a hydrogen atom, provided that when X¹ is a halogen atom, L¹ is a halogen atom having higher leaving ability than the halogen atom represented by X¹,
or a salt thereof, with a compound represented by the formula (III): Me₂(OH)C-C≡CH, in the presence of a metal catalyst and a base (1),
(b) a step of obtaining a compound represented by the formula (I-1") : 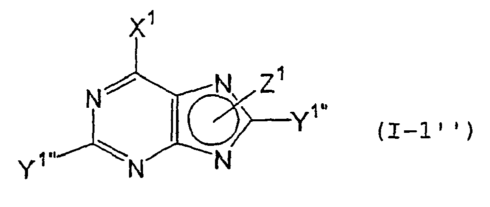 wherein
X¹ and Z¹ are as defined above, and
one of Y^{1"} is a group represented by the formula: HC≡C-, and the other Y^{1"} is a hydrogen atom, or a salt thereof, which comprises treating a compound of the formula (1-1') obtained in the step (a) or a salt thereof with a base (2), and
(c) a step of reacting a compound of the formula (I-1") obtained in the step (b) or a salt thereof, with a compound represented by the formula (IV): A-X wherein A is an aryl group optionally having substituents or a heterocyclic group optionally having substituents, and X is a halogen atom, in the presence of a metal catalyst and a base (1).

11. A production method of a compound represented by the formula (I-2') : 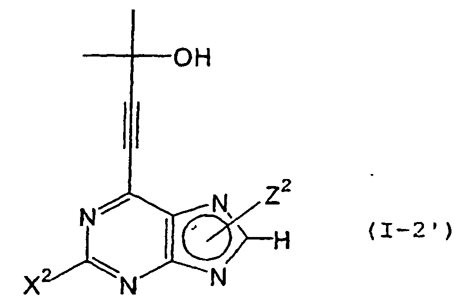 wherein
X² is an alkyl group, an alkoxy group, an aryl group, an optionally protected amino group, a halogen atom or a hydrogen atom, and
Z² is an alkyl group, an amino-protecting group or a hydrogen atom, which is attached to a nitrogen atom at the 7- or 9-position of the purine nucleus,
or a salt thereof, which comprises reacting a compound represented by the formula (II-2): 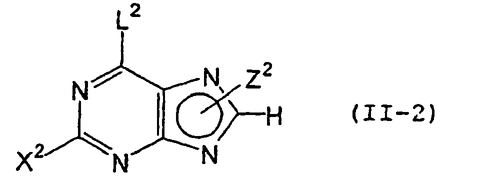
wherein
X² and Z² are as defined above, and
L² is a halogen atom, provided that when X² is a halogen atom,
L² is a halogen atom having higher leaving ability than the halogen atom represented by X², or the same halogen atom as X², or a salt thereof, with a compound represented by the formula (III) : Me₂(OH)C-C≡CH, in the presence of a metal catalyst and a base (1).

12. A production method of a compound represented by the formula (I-2"): 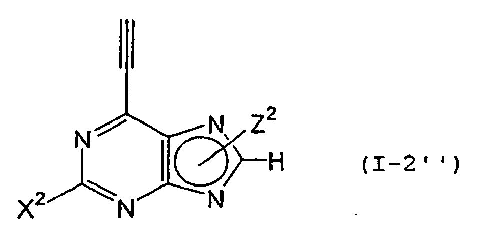 wherein
X² is an alkyl group, an alkoxy group, an aryl group, an optionally protected amino group, a halogen atom or a hydrogen atom, and
Z² is an alkyl group, an amino-protecting group or a hydrogen atom, which is attached to a nitrogen atom at the 7- or 9-position of the purine nucleus,
or a salt thereof, which comprises treating a compound represented by the formula (I-2'): 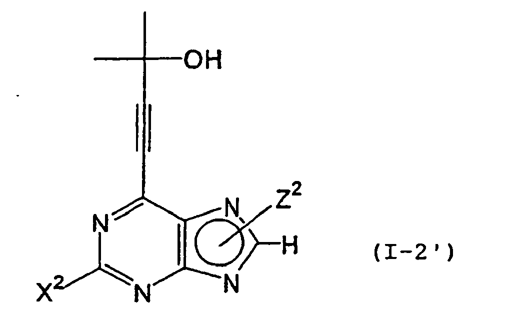
wherein
X² and Z² are as defined above,
or a salt thereof, with a base (2).

13. A production method of a compound represented by the formula (I-2"'): 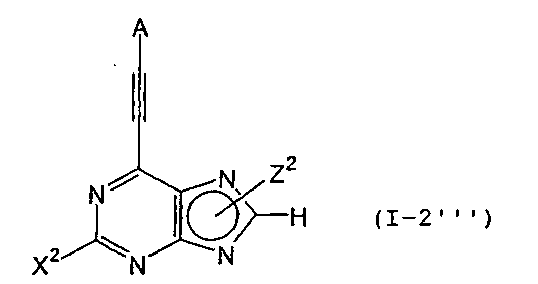 wherein
A is an aryl group optionally having substituents or a heterocyclic group optionally having substituents,
X² is an alkyl group, an alkoxy group, an aryl group, an optionally protected amino group, a halogen atom or a hydrogen atom, and
Z² is an alkyl group, an amino-protecting group or a hydrogen atom, which is attached to a nitrogen atom at the 7- or 9-position of the purine nucleus,
or a salt thereof, which comprises reacting a compound represented by the formula (I-2"): 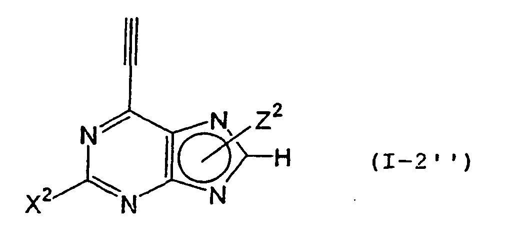
wherein
X² and Z² are as defined above,
or a salt thereof, with a compound represented by the formula (IV): A-X, wherein A is as defined above, and X is a halogen atom, in the presence of a metal catalyst and a base (1).

14. A production method of a compound represented by the formula (I-2"'): 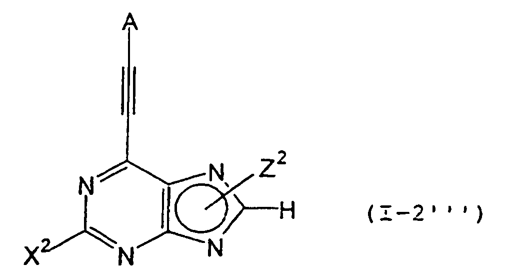 wherein
A is an aryl group optionally having substituents or a heterocyclic group optionally having substituents,
X² is an alkyl group, an alkoxy group, an aryl group, an optionally protected amino group, a halogen atom or a hydrogen atom, and
Z² is an alkyl group, an amino-protecting group or a hydrogen atom, which is attached to a nitrogen atom at the 7- or 9-position of the purine nucleus,
or a salt thereof, which comprises the following steps (a)-(c) :
(a) a step of obtaining a compound represented by the formula (I-2'): 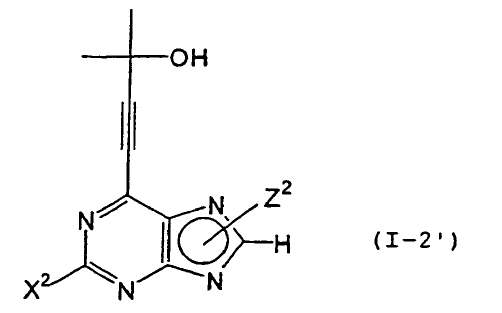 wherein
X² is an alkyl group, an alkoxy group, an aryl group, an optionally protected amino group, a halogen atom or a hydrogen atom, and
Z² is an alkyl group, an amino-protecting group or a hydrogen atom, which is attached to a nitrogen atom at the 7- or 9-position of the purine nucleus,
or a salt thereof, which comprises reacting a compound represented by the formula (II-2): 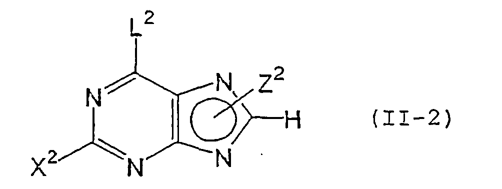
wherein
X² and Z² are as defined above, and
L² is a halogen atom, provided that when X² is a halogen atom,
L² is a halogen atom having higher leaving ability than the halogen atom represented by X², or the same halogen atom as X², or a salt thereof, with a compound represented by the formula (III): Me₂(OH)C-C≡CH, in the presence of a metal catalyst and a base (1),
(b) a step of obtaining a compound represented by the formula (I-2"): 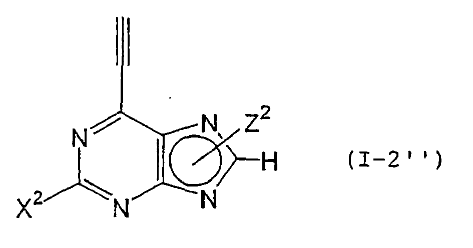
wherein
X² and Z² are as defined above,
or a salt thereof, which comprises treating a compound of the formula (I-2') obtained in the step (a) or a salt thereof, with a base (2), and
(c) a step of reacting a compound of the formula (I-2") obtained in the step (b) or a salt thereof, with a compound represented by the formula (IV): A-X, wherein A is an aryl group optionally having substituents or a heterocyclic group optionally having substituents, and X is a halogen atom, in the presence of a metal catalyst and a base (1).

15. The production method of any of claims 9, 10, 11, 13 and 14, wherein the metal catalyst is a palladium compound.

16. The production method of any of claim 9, 10, 11, 13 and 14, wherein the metal catalyst is a combination of a palladium compound and a copper compound.

17. The production method of claim 15 or 16, wherein the palladium compound is bis(triphenylphosphine)palladium dichloride or tetrakis(triphenylphosphine)palladium.

18. The production method of claim 16, wherein the copper compound is at least one selected from cuprous iodide, cuprous bromide and cuprous chloride.

19. The production method of any of claims 9, 10, 11, 13 and 14-18, wherein the base (1) is an amine compound.

20. The production method of claim 19, wherein the amine compound is trialkylamine.

21. The production method of claim 20, wherein the trialkylamine is triethylamine or ethyldiisopropylamine.

22. The production method of any of claims 8, 10, 12 and 14-21, wherein the base (2) is alkali metal hydroxide or alkali metal carbonate.

## Patentansprüche

1. Verbindung, wiedergegeben durch die Formel (I-1): 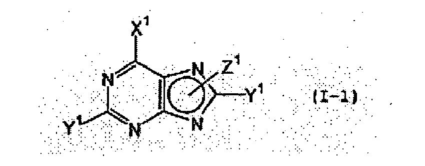 worin
X¹ eine Alkylgruppe ist, eine Alkoxygruppe, eine Arylgruppe, eine optional geschützte Aminogruppe, ein Halogenatom oder ein Wasserstoffatom,
eine von Y¹ eine Gruppe ist, wiedergegeben durch die Formel: R-C≡C-, worin R ein Wasserstoffatom ist und der andere Y¹-Gruppe ein Wasserstoffatom, und
Z¹ eine Alkylgruppe ist, eine Aminoschutzgruppe oder ein Wasserstoffatom, welche an ein Stickstoffatom an der 7- oder 9-Position des Purinkerns gebunden ist,
oder ein Salz derselben.

2. Verbindung nach Anspruch 1, worin X¹ ein Halogenatom ist und Z¹ eine Aminoschutzgruppe oder ein Wasserstoffatom.

3. Verbindung nach Anspruch 1 oder 2, worin X¹ ein Chloratom ist.

4. Verbindung nach einem der Ansprüche 1 bis 3, worin Z¹ Tetrahydropyran-2-yl, Benzyl oder ein Wasserstoffatom ist.

5. Verbindung, wiedergegeben durch die Formel (I-2): 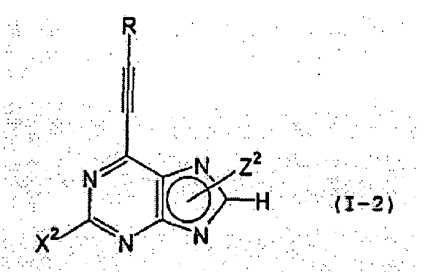 worin
R ein Wasserstoffatom ist oder eine Kohlenwasserstoffgruppe mit optionalen Substituenten,
X² eine Alkylgruppe ist, eine Alkoxygruppe, eine Arylgruppe, eine optional geschützte Aminogruppe oder ein Halogenatom, und
Z² eine Alkylgruppe ist, eine Aminoschutzgruppe oder ein Wasserstoffatom, welche an ein Stickstoffatom an der 7- oder 9-Position des Purinkerns gebunden ist,
oder ein Salz derselben.

6. Verbindung nach Anspruch 5, worin R ein Wasserstoffatom ist oder Me₂(OH)C-, X² eine optional geschützte Aminogruppe und Z² eine Aminoschutzgruppe oder ein Wasserstoffatom.

7. Verbindung nach Anspruch 5 oder 6, worin Z² Tetrahydropyran-2-yl, Benzyl oder ein Wasserstoffatom ist.

8. Verfahren zur Herstellung einer Verbindung, wiedergegeben durch die Formel (I-1"): 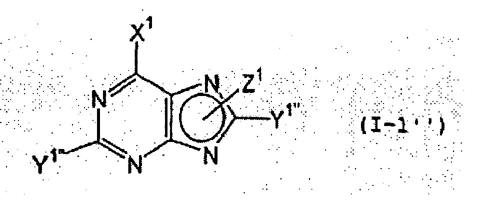 worin
X¹ eine Alkylgruppe ist, eine Alkoxygruppe, eine Arylgruppe, eine optional geschützte Aminogruppe, ein Halogenatom oder ein Wasserstoffatom,
Z¹ eine Alkylgruppe ist, eine Aminoschutzgruppe oder ein Wasserstoffatom, welche an ein Stickstoffatom an der 7- oder 9-Position des Purinkerns gebunden ist, und einer der Y^{1"} ein Wasserstoffatom ist, oder ein Salz derselben,
wobei das Verfahren das Behandeln der Verbindung, wiedergegeben durch die Formel (I-1'): 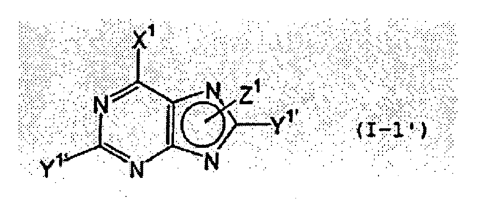
worin
X¹ und Z¹ wie oben definiert sind, und
einer der Y^{1'} eine Gruppe ist, wiedergegeben durch die Formel: Me₂(OH)C-C≡C-, und die andere Y^{1'}-Gruppe ein Wasserstoffatom ist,
oder ein Salz derselben mit einer Base (2).

9. Verfahren zur Herstellung einer Verbindung, wiedergegeben durch die Formel (I-1"'): 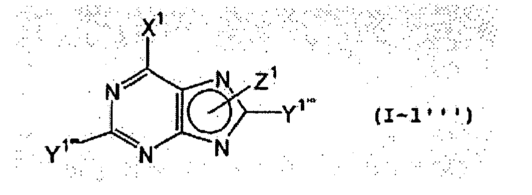 worin
X¹ eine Alkylgruppe ist, eine Alkoxygruppe, eine Arylgruppe, eine optional geschützte Aminogruppe, ein Halogenatom oder ein Wasserstoffatom,
Z¹ eine Alkylgruppe ist, eine Aminoschutzgruppe oder ein Wasserstoffatom, welche(s) an ein Stickstoffatom an der 7- oder 9-Position des Purinkerns gebunden ist, und
eine der Y^{1"'} eine Gruppe ist, wiedergegeben durch die Formel: A-C≡C-, worin A eine Arylgruppe ist, optional mit Substituenten, oder eine heterocyclische Gruppe, optional mit Substituenten, und die andere Y^{1"'}-Gruppe ein Wasserstoffatom ist,
oder ein Salz derselben, wobei das Verfahren das Umsetzen einer Verbindung, wiedergegeben durch die Formel (I-1"): 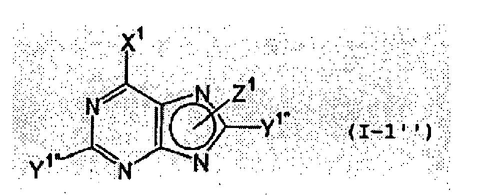
worin
X¹ und Z¹ wie oben definiert sind, und
eine von Y^{1"} eine Gruppe ist, wiedergegeben durch die Formel: HC≡C-, und die andere Y^{1"}-Gruppe ein Wasserstoffatom ist,
oder ein Salz derselben, mit einer Verbindung, wiedergegeben durch die Formel (IV): A-X, wobei A wie oben definiert und X ein Halogenatom ist, in der Gegenwart eine Metallkatalysators und einer Base (1) umfasst.

10. Verfahren zur Herstellung einer Verbindung, wiedergegeben durch die Formel (I-1"'): 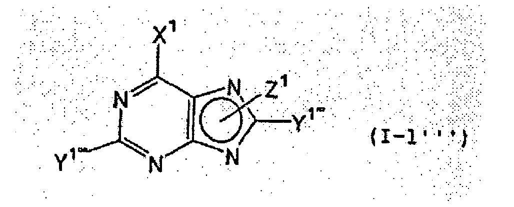 worin
X¹ eine Alkylgruppe ist, eine Alkoxygruppe, eine Arylgruppe, eine optional geschützte Aminogruppe, a Halogenatom oder ein Wasserstoffatom,
eine der Y^{1"'} eine Gruppe ist, wiedergegeben durch die Formel: A-C≡C-, worin A eine Arylgruppe ist, optional mit Substituenten, oder eine heterocyclische Gruppe, optional mit Substituenten, und die andere Y^{1"'}-Gruppe ein Wasserstoffatom ist, und
Z¹ eine Alkylgruppe ist, eine Aminoschutzgruppe oder ein Wasserstoffatom, welche an ein Stickstoffatom an der 7- oder 9-Position des Purinkerns gebunden ist,
oder ein Salz derselben, wobei das Verfahren die folgenden Schritte (a) - (c) umfasst:
(a) einen Schritt des Gewinnens einer Verbindung, wiedergegeben durch die Formel (I-1'): 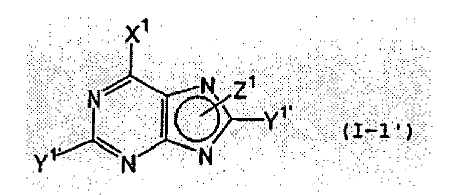 worin
X¹ eine Alkylgruppe ist, eine Alkoxygruppe, eine Arylgruppe, eine optional geschützte Aminogruppe, ein Halogenatom oder ein Wasserstoffatom,
Z¹ eine Alkylgruppe ist, eine Aminoschutzgruppe oder ein Wasserstoffatom, welche(s) an ein Stickstoffatom an der 7- oder 9-Position des Purinkerns gebunden ist, und
eine von Y^{1'} eine Gruppe ist, wiedergegeben durch die Formel: Me₂(OH)C-C≡C-, und die andere Gruppe Y^{1'} ein Wasserstoffatom ist,
oder ein Salz derselben, welches das Umsetzen einer Verbindung, wiedergegeben durch die Formel (II-1):
**Formel (II-1) einfügen**
umfasst, worin
X¹ und Z¹ wie oben definiert sind, und
eine von L¹ ein Halogenatom ist und die andere Gruppe L¹ ein Wasserstoffatom, vorausgesetzt dass, wenn X¹ ein Wasserstoffatom ist, L¹ ein Halogenatom mit größerer Abgangsfähigkeit ist als das durch X¹ wiedergegebene Halogenatom, oder ein Salz derselben, mit einer Verbindung, wiedergegeben durch die Formel (III): Me₂(OH)C-C≡CH, in der Gegenwart eines Metallkatalysators und einer Base (1),
(b) einen Schritt des Gewinnens einer Verbindung, wiedergegeben durch die Formel (I-1"):
**Formel (I-1") einfügen**
worin
X¹ und Z¹ wie oben definiert sind, und
eine von Y^{1"} eine Gruppe ist, wiedergegeben durch die Formel: HC≡C-, und die andere Gruppe Y^{1"} ist ein Wasserstoffatom, oder ein Salz derselben, welcher das Behandeln einer in Schritt (a) erhaltenen Verbindung der Formel (I-1') oder eines Salzes derselben mit einer Base (2) umfasst, und
(c) einen Schritt des Umsetzens einer Verbindung der Formel (I-1"), erhalten in Schritt (b), oder eines Salzes derselben, mit einer durch die Formel (IV): A-X wiedergegebenen Verbindung, worin A eine Arylgruppe, optional mit Substituenten, oder eine heterocyclische Gruppe, optional mit Substituenten, und X ein Halogenatom ist, in der Gegenwart eines Metallkatalysators und einer Base (1).

11. Verfahren zur Herstellung einer Verbindung, wiedergegeben durch die Formel (I-2'): 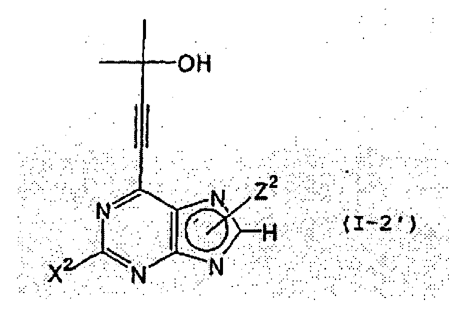 worin
X² eine Alkylgruppe ist, eine Alkoxygruppe, eine Arylgruppe, eine optional geschützte Aminogruppe, ein Halogenatom oder ein Wasserstoffatom, und
Z² eine Alkylgruppe ist, eine Aminoschutzgruppe oder ein Wasserstoffatom, welche an ein Stickstoffatom an der 7- oder 9-Position des Purinkerns gebunden ist,
oder ein Salz derselben, wobei das Verfahren ein Umsetzten der Verbindung umfasst, wiedergegeben durch die Formel (II-2'): 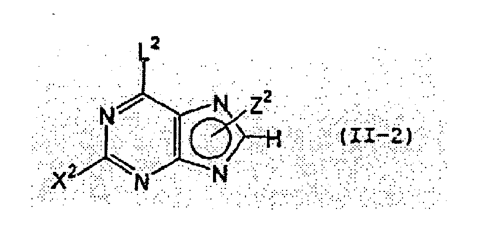
worin
X² und Z² wie oben definiert sind, und
L² ein Halogenatom ist, unter der Vorraussetzung dass, wenn X² ein Halogenatom ist, L² ein Halogenatom mit einem größeren Abgangsvermögen als das durch X² wiedergegebene Halogenatom ist, oder das gleiche Halogenatom wie X² ist, oder ein Salz derselben, mit einer Verbindung, wiedergegeben durch die Formel (III): Me₂C-C≡CH, in der Gegenwart eines Metallkatalysators und einer Base (1).

12. Verfahren zur Herstellung einer Verbindung, wiedergegeben durch die Formel (I-2"): 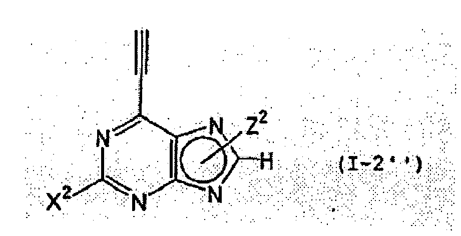 worin
X² eine Alkylgruppe ist, eine Alkoxygruppe, eine Arylgruppe, eine optional geschützte Aminogruppe, ein Halogenatom oder ein Wasserstoffatom, und
Z² eine Alkylgruppe ist, eine Aminoschutzgruppe oder ein Wasserstoffatom, welche an ein Stickstoffatom an der 7- oder 9-Position des Purinkerns gebunden ist,
oder ein Salz derselben, wobei das Verfahren ein Umsetzten der Verbindung, wiedergegeben durch die Formel (I-2'): 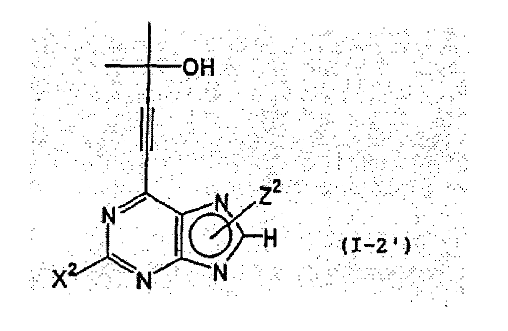
worin
X² und Z² wie oben definiert sind,
oder ein Salz derselben mit einer Base (2) umfasst.

13. Verfahren zur Herstellung einer Verbindung, wiedergegeben durch die Formel (I-2"'): 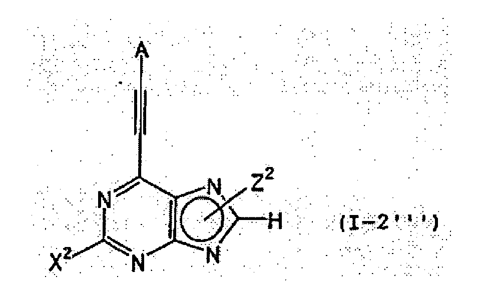 worin
A eine Arylgruppe ist, optional mit Substituenten, oder eine heterocyclische Gruppe, optional mit Substituenten,
X² eine Alkylgruppe ist, eine Alkoxygruppe, eine Arylgruppe, eine optional geschützte Aminogruppe, ein Halogenatom oder ein Wasserstoffatom, und
Z² eine Alkylgruppe ist, eine Aminoschutzgruppe oder ein Wasserstoffatom, welche an ein Stickstoffatom an der 7- oder 9-Position des Purinkerns gebunden ist,
oder ein Salz derselben, wobei das Verfahren ein Umsetzten der Verbindung umfasst, wiedergegeben durch die Formel (I-2"): 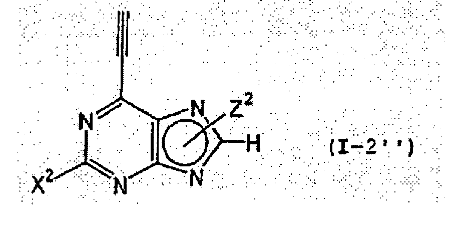
worin
X² und Z² wie oben definiert sind,
oder ein Salz derselben mit einer Verbindung, wiedergegeben durch die Formel (IV): A-X, wobei A wie oben definiert ist und X ein Halogenatom, in der Gegenwart eines Metallkatalysators und einer Base (1) umfasst.

14. Verfahren zur Herstellung einer Verbindung, wiedergegeben durch die Formel (I-2"'): 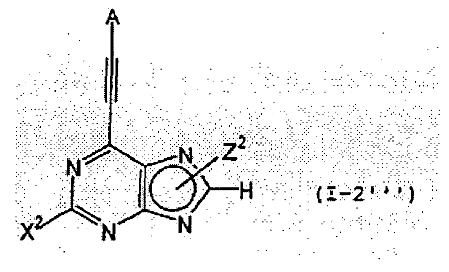 worin
A eine Arylgruppe ist, optional mit Substituenten, oder eine heterocyclische Gruppe, optional mit Substituenten,
X² eine Alkylgruppe ist, eine Alkoxygruppe, eine Arylgruppe, eine optional geschützte Aminogruppe, a Halogenatom oder ein Wasserstoffatom, und
Z² eine Alkylgruppe ist, eine Aminoschutzgruppe oder ein Wasserstoffatom, welche an ein Stickstoffatom an der 7- oder 9-Position des Purinkerns gebunden ist,
oder ein Salz derselben, wobei das Verfahren die folgenden Schritte (a) bis (c) umfasst:
(a) einen Schritt des Gewinnens einer Verbindung, wiedergegeben durch die Formel (I-2'): 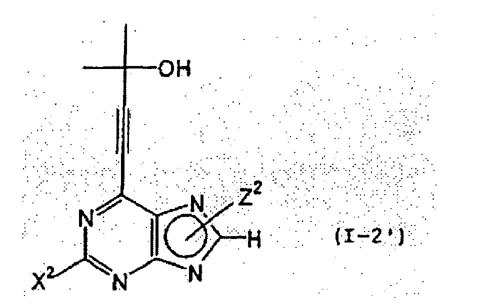 worin
X² eine Alkylgruppe ist, eine Alkoxygruppe, eine Arylgruppe, eine optional geschützte Aminogruppe, a Halogenatom oder ein Wasserstoffatom, und
Z² eine Alkylgruppe ist, eine Aminoschutzgruppe oder ein Wasserstoffatom, welche(s) an ein Stickstoffatom an der 7- oder 9-Position des Purinkerns gebunden ist,
oder ein Salz derselben, welches ein Umsetzen einer Vernindung, wiedergegeben durch die Formel (II-2):
**Formel (II-2) einfügen**
umfasst, worin
X² und Z² wie oben definiert sind, und
L² ein Halogenatom ist, vorausgesetzt dass, wenn X² ein Halogenatom ist, L² ein Halogenatom mit einem größeren Abgangsvermögen als das durch X² wiedergegebene Halogenatom ist, oder dasselbe Halogenatom wie X², oder ein Salz derselben, mit einer Verbindung, wiedergegeben durch die Formel (III): Me₂(OH)C-C≡CH, in der Gegenwart eines Metallkatalysators und einer Base (1),
(b) einen Schritt des Gewinnens einer Verbindung, wiedergegeben durch die Formel (II-2"):
**Formel (II-2") einfügen**
worin
X² und Z² wie oben definiert sind,
oder ein Salz derselben, welches das Behandeln einer Verbindung der Formel (I-2'), erhalten in Schritt (a), oder eines Salzes derselben mit einer Base (2) umfasst, und
(c) einen Schritt des Umsetzens einer Verbindung der Formel (I-2"), erhalten in Schritt (b), oder eines Salzes derselben mit einer Verbundung, wiedergegeben durch die Formel (IV): A-X, worin A eine Arylgruppe ist, optional mit Substituenten, oder eine heterocyclische Gruppe, optional mit Substituenten, und X ein Halogenatom in der Gegenwart eines Metallkatalysators und einer Base (1).

15. Verfahren nach einem der Ansprüche 9, 10, 11, 13 and 14, wobei der Metallkatalysator einer Palladiumverbindung ist.

16. Verfahren nach einem der Ansprüche 9, 10, 11, 13 und 14, wobei der Metallkatalysator eine Kombination einer Palladiumverbindung und einer Kupferverbindung ist.

17. Verfahren nach Anspruch 15 oder 16, wobei die Palladiumverbindung Bis(triphenylphosphin)palladiumdichlorid oder Tetrakis(triphenlyphosphin)-palladium ist.

18. Verfahren nach Anspruch 16, wobei die Kupferverbindung wenigstens eine ist, ausgewählt aus Kupfer(I)jodid, Kupfer(I)bromid und Kupfer(I)chlorid.

19. Verfahren nach einem der Ansprüche 9, 10, 11, 13 und 14 bis 18, wobei die Base (1) eine Aminverbindung ist.

20. Verfahren nach Anspruch 19, wobei die Aminverbindung Trialkylamin ist.

21. Verfahren nach Anspruch 20, wobei das Trialkylamin Triethylamin oder Ethyldiisopropylamin ist.

22. Verfahren nach einem der Ansprüche 8, 10, 12 und 14 bis 21, wobei die Base (2) ein Alkylimetallhydroxid oder Alkalimetallcarbonat ist.

## Revendications

1. Composé représenté par la formule (I - 1) : 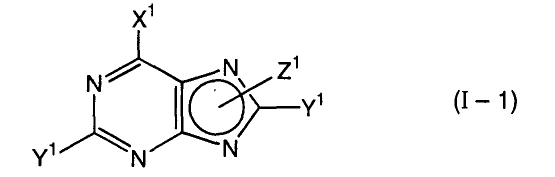 dans laquelle
X¹ est un groupe alkyle, un groupe alcoxy, un groupe aryle, un groupe amino éventuellement protégé, un atome d'halogène ou un atome d'hydrogène,
un de Y¹ est un groupe représenté par la formule : R-C≡C- dans laquelle R est atome d'hydrogène,
et l'autre Y¹ est un atome d'hydrogène, et
Z¹ est un groupe alkyle, un groupe protecteur du groupe amino, ou un atome d'hydrogène, qui est lié à l'atome d'azote en position 7 ou 9 du noyau purine,
ou un sel de celui-ci.

2. Composé selon la revendication 1, dans lequel X¹ est un atome d'halogène et Z¹ est un groupe protecteur du groupe amino.

3. Composé selon la revendication 1 ou 2, dans lequel X¹ est un atome de chlore.

4. Composé selon l'une des revendications 1 à 3, dans lequel Z¹ est un groupe tétrahydropyran-2-yle, benzyle ou un atome d'hydrogène.

5. Composé représenté par la formule (I-2) : 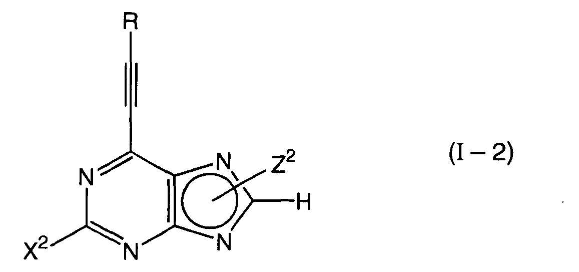 dans laquelle
R est un atome d'hydrogène ou un groupe hydrocarboné ayant éventuellement des substituants,
X² est un groupe alkyle, un groupe alcoxy, un groupe aryle, un groupe amino éventuellement protégé ou un atome d'halogène,
Z² est un groupe alkyle, un groupe protecteur du groupe amino ou un atome d'hydrogène, qui est lié à un atome d'azote en position 7 ou 9 du noyau purine,
ou un sel de celui-ci.

6. Composé selon la revendication 5, dans lequel R est un atome d'hydrogène ou Me₂(OH)C-, X² est un groupe amino éventuellement protégé, et Z² est un groupe protecteur du groupe amino ou un atome d'hydrogène.

7. Composé selon la revendication 5 ou 6, dans lequel Z² est un groupe tétrahydropyran-2-yle, benzyle ou un atome d'hydrogène.

8. Procédé de production d'un composé représenté par la formule (I-1") : 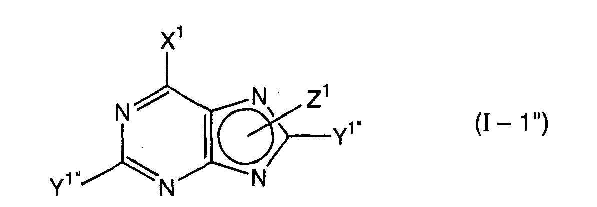 dans laquelle
X¹ est un groupe alkyle, un groupe alcoxy, un groupe aryle, un groupe amino éventuellement protégé, un atome d'halogène ou un atome d'hydrogène,
Z¹ est un groupe alkyle, un groupe protecteur du groupe amino ou un atome d'hydrogène, qui est lié à un atome d'azote en position 7 ou 9 du noyau purine, et
un de Y^{1"} est un groupe représenté par la formule : H-C≡C-, et l'autre Y^{1"} est un atome d'hydrogène, ou un sel de celui-ci, qui comprend le traitement d'un composé représenté par la formule (I-1') : 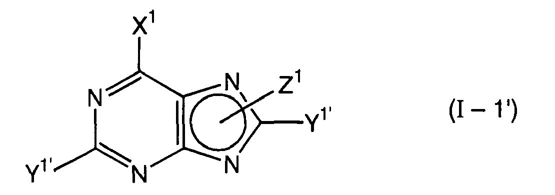 dans laquelle
X¹ et Z¹ sont tels que définis ci-dessus, et
un de Y^{1'} est un groupe représenté par la formule : Me₂(OH)C-C≡C-, et l'autre Y^{1'} est un atome d'hydrogène,
ou un sel de celui-ci, avec une base (2).

9. Procédé de production d'un composé représenté par la formule (I-1"'): 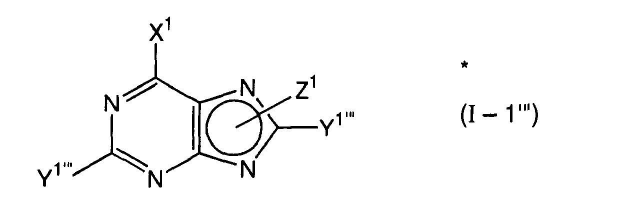 dans laquelle
X¹ est un groupe alkyle, un groupe alcoxy, un groupe aryle, un groupe amino éventuellement protégé, un atome d'halogène ou un atome d'hydrogène,
Z¹ est un groupe alkyle, un groupe protecteur du groupe amino ou un atome d'hydrogène, qui est lié à un atome d'azote en position 7 ou 9 du noyau purine, et
un de Y^{1"'} est un groupe représenté par la formule : A-C≡C-, dans laquelle A est un groupe aryle ayant éventuellement des substituants ou un groupe hétérocyclique ayant éventuellement des substituants, et l'autre Y^{1'"} est un atome d'hydrogène,
ou un sel de celui-ci, qui comprend la réaction d'un composé représenté par la formule (I-1") : 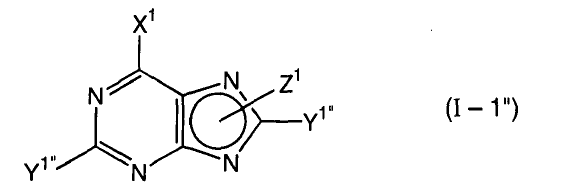 dans laquelle
X¹ et Z¹ sont tels que définis ci-dessus, et
un de Y^{1"} est un groupe représenté par la formule : HC≡C-, et l'autre Y1" est un atome d'hydrogène,
ou un sel de celui-ci, avec un composé représenté par la formule (IV) :
A-X dans laquelle A est tel que défini ci-dessus, et X est un atome d'halogène, en présence d'un catalyseurs métallique et d'une base (1).

10. Procédé de production d'un composé représenté par la formule (I-1'"): 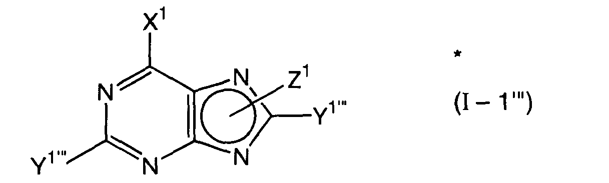 dans laquelle
X¹ est un groupe alkyle, un groupe alcoxy, un groupe aryle, un groupe amino éventuellement protégé, un atome d'halogène ou un atome d'hydrogène,
un de Y^{1'"} est un groupe représenté par la formule : A-C≡C-, dans laquelle A est un groupe aryle ayant éventuellement des substituants ou un groupe hétérocyclique ayant éventuellement des substituants, et l'autre Y^{1'"} est un atome d'hydrogène, et
Z¹ est un groupe alkyle, un groupe protecteur du groupe amino ou un atome d'hydrogène, qui est lié à un atome d'azote en position 7 ou 9 du noyau purine,
ou un sel de celui-ci, qui comprend les étapes suivantes (a) à (c) :
(a) une étape d'obtention d'un composé représenté par la formule (I-1'): 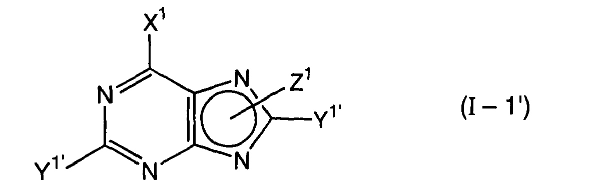 dans laquelle
X¹ est un groupe alkyle, un groupe alcoxy, un groupe aryle, un groupe amino éventuellement protégé, un atome d'halogène ou un atome d'hydrogène,
Z¹ est un groupe alkyle, un groupe protecteur du groupe amino ou un atome d'hydrogène, qui est lié à un atome d'azote en position 7 ou 9 du noyau purine, et
un de Y^{1'} est un groupe représenté par la formule : Me₂(OH)C-C≡C-, et l'autre Y^{1'} est un atome d'hydrogène,
ou un sel de celui-ci, qui comprend la réaction d'un composé représenté par la formule (II-1) : 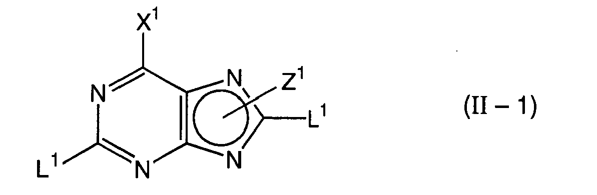
dans laquelle
X¹ et Z¹ sont tels que définis ci-dessus, et
un de L¹ est un atome d'halogène, et l'autre L¹ est un atome d'hydrogène, à condition que quand X¹ est un atome d'halogène, L¹ soit un atome d'halogène ayant une aptitude à partir supérieure à l'atome d'halogène représenté par X¹,
ou un sel de celui-ci, avec un composé représenté par la formule (III) : Me₂(OH)C-C≡CH, en présence d'un catalyseur métallique et d'une base (1),
(b) une étape d'obtention d'un composé représenté par la formule (I-1") : 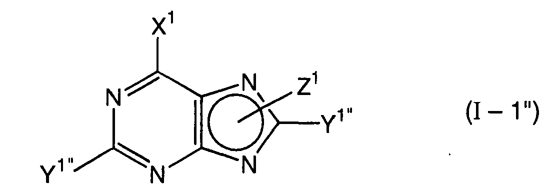
dans laquelle
X¹ et Z¹ sont tels que définis ci-dessus, et
un de Y^{1"} est un groupe représenté par la formule : HC≡C-, et l'autre Y^{1"} est un atome d'hydrogène, ou un sel de celui-ci, qui comprend le traitement d'un composé de formule (I-1') obtenu dans l'étape (a) ou un sel de celui-ci avec une base (2), et
(c) une étape de réaction d'un composé de formule (I-1") obtenu dans l'étape (b) ou un sel de celui-ci, avec un composé représenté par la formule (IV) : A-X dans laquelle A est un groupe aryle ayant éventuellement des substituants ou un groupe hétérocyclique ayant éventuellement des substituants, et X est un atome d'halogène, en présence d'un catalyseur métallique et d'une base (1).

11. Procédé de production d'un composé représenté par la formule (I-2') : 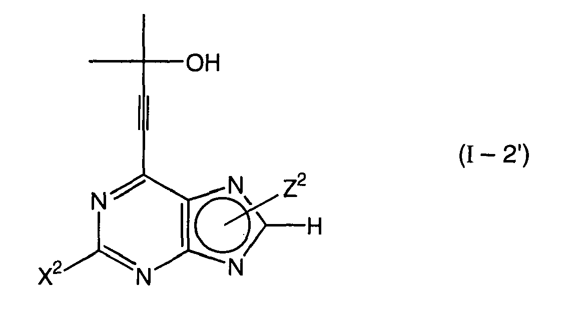 dans laquelle
X² est un groupe alkyle, un groupe alcoxy, un groupe aryle, un groupe amino éventuellement protégé, un atome d'halogène ou un atome d'hydrogène,
Z² est un groupe alkyle, un groupe protecteur du groupe amino ou un atome d'hydrogène, qui est lié à un atome d'azote en position 7 ou 9 du noyau purine,
ou un sel de celui-ci, qui comprend la réaction d'un composé représenté par la formule (II-2) : 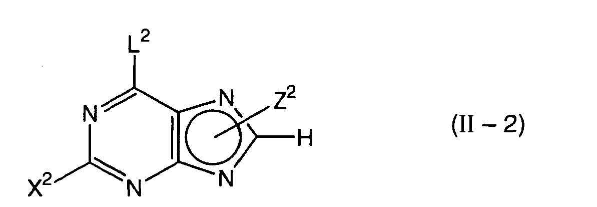
dans laquelle
X² et Z² sont tels que définis ci-dessus, et
L² est un atome d'halogène, à condition que quand X² est un atome d'halogène, L² soit un atome d'halogène ayant une aptitude à partir supérieure à l'atome d'halogène représenté par X², ou le même atome d'halogène que X², ou un sel de celui-ci, avec un composé représenté par la formule (III) : Me₂(OH)C-C≡CH, en présence d'un catalyseur métallique et d'une base (1).

12. Procédé de production d'un composé représenté par la formule (I-2") : 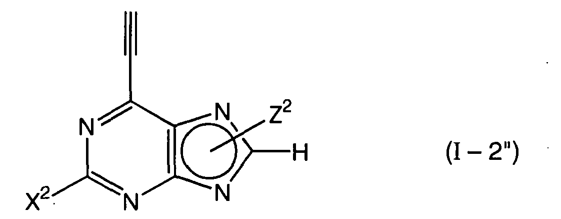 dans laquelle
X² est un groupe alkyle, un groupe alcoxy, un groupe aryle, un groupe amino éventuellement protégé, un atome d'halogène ou un atome d'hydrogène, et
Z² est un groupe alkyle, un groupe protecteur du groupe amino ou un atome d'hydrogène, qui est lié à un atome d'azote en position 7 ou 9 du noyau purine,
ou un sel de celui-ci, qui comprend le traitement d'un composé représenté par la formule (I-2') : 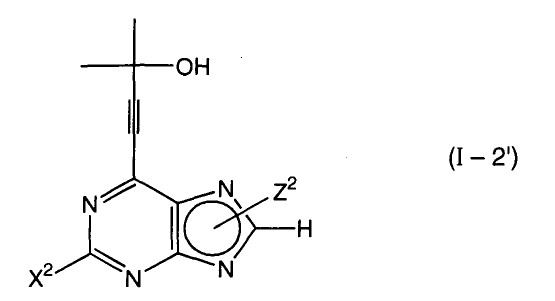
dans laquelle
X² et Z² sont tels que définis ci-dessus,
ou un sel de celui-ci, avec une base (2).

13. Procédé de production d'un composé représenté par la formule (I-2"') : 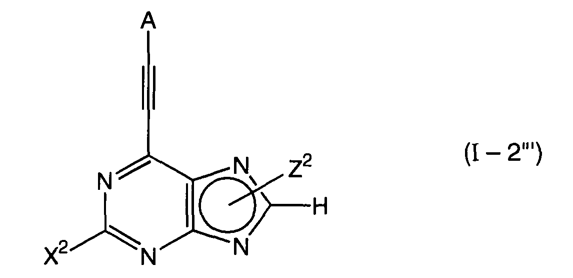 dans laquelle
A est un groupe aryle ayant éventuellement des substituants ou un groupe hétérocyclique ayant éventuellement des substituants,
X² est un groupe alkyle, un groupe alcoxy, un groupe aryle, un groupe amino éventuellement protégé, un atome d'halogène ou un atome d'hydrogène, et
Z² est un groupe alkyle, un groupe protecteur du groupe amino ou un atome d'hydrogène, qui est lié à un atome d'azote en position 7 ou 9 du noyau purine,
ou un sel de celui-ci, qui comprend la réaction d'un composé représenté par la formule (I-2") : 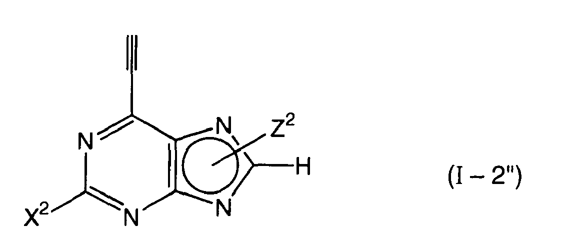
dans laquelle
X² et Z² sont tels que définis ci-dessus,
ou un sel de celui-ci, avec un composé représenté par la formule (IV) : A-X, dans laquelle A est tel que défini ci-dessus, et X est un atome d'halogène, en présence d'un catalyseur métallique et d'une base (1).

14. Procédé de production d'un composé représenté par la formule (I-2"') : 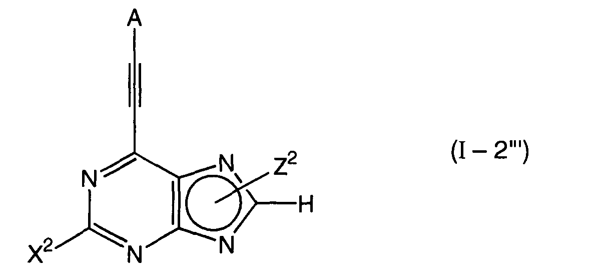 dans laquelle
A est un groupe aryle ayant éventuellement des substituants ou un groupe hétérocyclique ayant éventuellement des substituants,
X² est un groupe alkyle, un groupe alcoxy, un groupe aryle, un groupe amino éventuellement protégé, un atome d'halogène ou un atome d'hydrogène, et
Z² est un groupe alkyle, un groupe protecteur du groupe amino ou un atome d'hydrogène, qui est lié à un atome d'azote en position 7 ou 9 du noyau purine,
ou un sel de celui-ci, qui comprend les étapes suivantes (a) à (c) :
(a) une étape d'obtention d'un composé représenté par la formule (I-2') 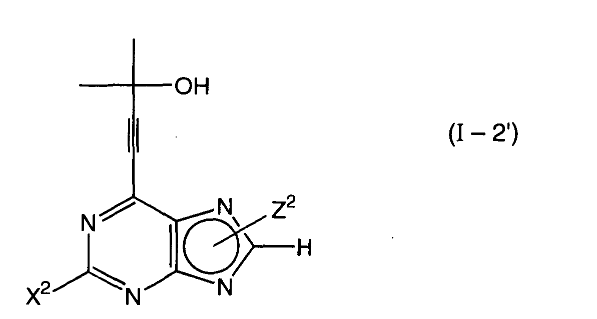 dans laquelle
X² est un groupe alkyle, un groupe alcoxy, un groupe aryle, un groupe amino éventuellement protégé, un atome d'halogène ou un atome d'hydrogène,
Z² est un groupe alkyle, un groupe protecteur du groupe amino ou un atome d'hydrogène, qui est lié à un atome d'azote en position 7 ou 9 du noyau purine, et
ou un sel de celui-ci, qui comprend la réaction d'un composé représenté par la formule (II-2) : 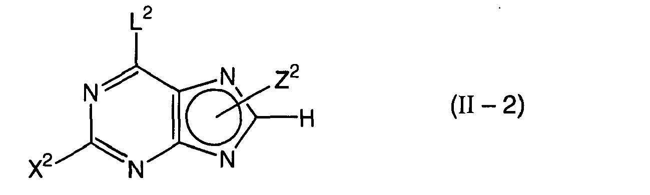
dans laquelle
X² et Z² sont tels que définis ci-dessus, et
L² est un atome d'halogène, à condition que quand X² est un atome d'halogène, L² soit un atome d'halogène ayant une aptitude à partir supérieure à l'atome d'halogène représenté par X², ou le même atome d'halogène que X²,
ou un sel de celui-ci, avec un composé représenté par la formule (III) : Me₂(OH)C-C≡CH, en présence d'un catalyseur métallique et d'une base (1),
(b) une étape d'obtention d'un composé représenté par la formule (I-2") 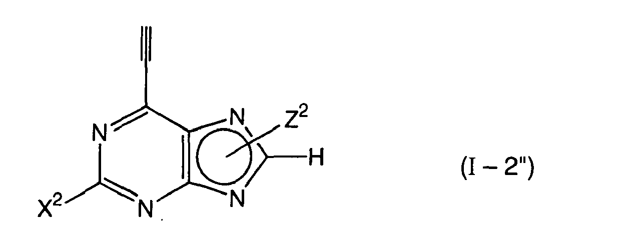 dans laquelle
X² et Z² sont tels que définis ci-dessus,
ou un sel de celui-ci, qui comprend le traitement d'un composé de formule (I-2') obtenu dans l'étape (a) ou un sel de celui-ci, avec une base (2), et
(c) une étape de réaction d'un composé de formule (I-2") obtenu dans l'étape (b) ou un sel de celui-ci, avec un composé représenté par la formule (IV) : A-X dans laquelle A est un groupe aryle ayant éventuellement des substituants ou un groupe hétérocyclique ayant éventuellement des substituants, et X est un atome d'halogène, en présence d'un catalyseur métallique et d'une base (1).

15. Procédé de production selon l'une quelconque des revendications 9, 10, 11, 13 et 14, dans lequel le catalyseur métallique est un composé de palladium.

16. Procédé de production selon l'une quelconque des revendications 9, 10, 11, 13 et 14, dans lequel le catalyseur métallique est une combinaison d'un composé de palladium et d'un composé de cuivre.

17. Procédé de production selon l'une quelconque des revendications 15 ou 16, dans lequel le composé de palladium est le dichlorure de bis(triphénylphosphine)palladium ou le tétrakis(triphénylphosphine)palladium.

18. Procédé de production selon la revendication 16, dans lequel le composé de cuivre en est au moins un choisi parmi l'iodure cuivreux, le bromure cuivreux et le chlorure cuivreux.

19. Procédé de production selon l'une quelconque des revendications 9, 10, 11, 13 et 14 à 18, dans lequel la base (1) est un composé amine.

20. Procédé de production selon la revendication 19, dans lequel le composé amine est la trialkylamine.

21. Procédé de production selon la revendication 20, dans lequel la trialkylamine est la triéthylamine ou l'éthyldiisopropylamine.

22. Procédé de production selon l'une quelconque des revendications 8, 10, 12 et 14 à 21, dans lequel la base (2) est un hydroxyde de métal alcalin ou un carbonate de métal alcalin.
